# EUROPÄISCHE PATENTSCHRIFT

(11) **EP 3 735 405 B1**
(45) Veröffentlichungstag und Bekanntmachung des Hinweises auf die Patenterteilung: **24.11.2021**
(21) Anmeldenummer: 19700011.0
(22) Anmeldetag: 02.01.2019
(51) Int. Cl.: C07C 263/20, C07C 263/10, C07C 265/14

(54) **VERFAHREN ZUR HERSTELLUNG VON METHYLEN-DIPHENYLEN-DIISOCYANATEN UND POLYMETHYLEN-POLYPHENYLEN-POLYISOCYANATEN**
METHOD FOR THE PREPARATION OF METHYLENE DIPHENYLENE DIISOCYANATES AND POLYMETHYLENE POLYPHENYLENE POLYISOCYANATES
PROCÉDÉ DE PRÉPARATION DE MÉTHYLÈNE-DIPHÉNYLÈNE-DIISOCYANATES ET DE POLYMÉTHYLÈNE-POLYPHÉNYLÈNE-POLYISOCYANATES

(30) Priorität: 05.01.2018 EP 18150434
(43) Veröffentlichungstag der Anmeldung: 11.11.2020
(73) Patentinhaber: Covestro Intellectual Property GmbH & Co. KG, 51373 Leverkusen (DE)
(72) Erfinder: KNAUF, Thomas, 41542 Dormagen (DE); PLATHEN, Peter, 47829 Krefeld (DE); MANZEL, Dirk, 47447 Moers (DE); WERSHOFEN, Stefan, 41065 Mönchengladbach (DE)
(74) Vertreter: Levpat
(86) Internationale Anmeldenummer: PCT/EP2019/050026
(87) Internationale Veröffentlichungsnummer: WO 2019/134909

(56) Entgegenhaltungen:
- WO-A1-2010/095927
- WO-A1-2017/050776

## Beschreibung

Die vorliegende Erfindung betrifft ein Verfahren zur Herstellung von Methylen-Diphenylen-Diisocyanat und optional eines Gemisches aus Methylen-Diphenylen-Diisocyanat und Polymethylen-Polyphenylen-Polyisocyanat, bei dem in einem **Schritt α)** eine Methylen-Diphenylen-Diisocyanat und Nebenkomponenten enthaltende Fraktion bereitgestellt wird, optional durch einen **Schritt α.1),** der Abtrennung von Methylen-Diphenylen-Diisocyanat und Nebenkomponenten aus einer Methylen-Diphenylen-Diisocyanat und Polymethylen-Polyphenylen-Polyisocyanat enthaltenden Fraktion, und bei dem in einem **Schritt β)** die Methylen-Diphenylen-Diisocyanat und Nebenkomponenten enthaltende Fraktion einer Reinigung umfassend eine Isomerentrennung durch Destillation und/oder Kristallisation in zwei oder mehr Teilschritten unter Erhalt von zwei oder mehr Methylen-Diphenylen-Diisocyanat-Reinfraktionen und einer Nebenkomponenten-Fraktion unterzogen wird, wobei die in Schritt β) erhaltene Nebenkomponenten-Fraktion in einen der Teilschritte von Schritt β), in dem keine der Methylen-Diphenylen-Diisocyanat-Reinfraktionen aus Schritt β) als Destillat oder Kristallisat gewonnen wird, und/oder, insofern durchgeführt, in Schritt α.1), zurückgeführt wird.

Isocyanate (1) werden in großen Mengen hergestellt und dienen hauptsächlich als Ausgangsstoffe zur Herstellung von Polyurethanen. Ihre Herstellung erfolgt zumeist durch Umsetzung der entsprechenden Amine (2) mit Phosgen (3), wobei Phosgen im stöchiometrischen Überschuss eingesetzt wird. Die Umsetzung der Amine mit dem Phosgen kann sowohl in der Gasphase als auch in der Flüssigphase erfolgen, wobei die Reaktion diskontinuierlich oder kontinuierlich durchgeführt werden kann (W. Siefken, Liebigs Ann. 562, 75 - 106 (1949)). Verfahren zur Herstellung von organischen Isocyanaten aus primären Aminen und Phosgen sind bereits vielfach beschrieben worden; rein exemplarisch sei auf Ullmanns Encyklopädie der technischen Chemie, 4. Aufl. (1977), Band 13, S. 351 bis 353 sowie G. Wegener et. al., Applied Catalysis A: General 221 (2001), 303 - 335, Elsevier Science B.V., verwiesen. Von großtechnischem Interesse sind dabei sowohl aromatische Isocyanate wie beispielsweise Methylen-Diphenylen-Diisocyanat (fortan MMDI - "monomeres MDI"), Gemische aus MMDI und Polymethylen-Polyphenylen-Polyisocyanate (das sind die höheren Homologen des MMDI, fortan PMDI, "polymeres MDI"; Gemische aus MMDI und PMDI werden fortan summarisch als MDI bezeichnet) oder Toluylendiisocyanat (TDI) als auch aliphatische Isocyanate wie z. B. 1,5-Pentandiisocyanat (PDI), 1,6-Hexamethylendiisocyanat (HDI), oder Isophorondiisocyanat (IPDI). Daneben sind auch Isocyanate mit benzylischen Isocyanatgruppen bedeutsam, insbesondere sei hier Xylylendiisocyanat (XDI) erwähnt.

Die moderne großtechnische Herstellung von Isocyanaten erfolgt semi-kontinuierlich (diskontinuierliche Durchführung eines Teils der Herstellungsschritte, z. B. diskontinuierliche Reaktion und kontinuierliche Aufarbeitung) oder kontinuierlich (alle Schritte kontinuierlich).

Die Verfahrensführung in der Flüssigphase, üblicherweise als Flüssigphasenphosgenierung bezeichnet, zeichnet sich dadurch aus, dass die Reaktionsbedingungen so gewählt werden, dass zumindest die Reaktionskomponenten Amin, Roh-Isocyanat und Phosgen, bevorzugt jedoch sämtliche Edukte, Produkte und Reaktionszwischenprodukte, bei den gewählten Bedingungen flüssig in einem geeigneten Lösungsmittel vorliegen. Nach erfolgter Umsetzung wird eine Gasphase enthaltend das Koppelprodukt Chlorwasserstoff und nicht umgesetztes (weil überstöchiometrisch eingesetztes) Phosgen abgetrennt; dabei verbleibt das angestrebte Isocyanat zusammen mit dem Lösungsmittel weitestgehend in der Flüssigphase. Das rohe Isocyanat fällt also im Gemisch mit Lösungsmittel als Flüssigkeitsstrom an, der zur Gewinnung von Rein-Isocyanat (und zur Rückgewinnung von Lösungsmittel sowie gelösten Anteilen von Phosgen und Chlorwasserstoff) aufgearbeitet wird.

In allen großtechnisch relevanten Isocyanat-Herstellungsprozessen fällt demnach ein flüssiger Roh-Isocyanatstrom an, der zur Gewinnung des angestrebten Isocyanats in Reinform und zur Rückgewinnung anderer Wertstoffe wie Lösungsmittel aufgearbeitet werden muss. Diese Aufarbeitung umfasst im Allgemeinen die Abtrennung von Lösungsmittel, gelöstem Phosgen und gelöstem Chlorwasserstoff. Daran schließt sich eine Feinreinigung des Isocyanats an, die erforderlichenfalls auch eine Isomerentrennung umfassen kann. Abhängig von der Art des Isocyanats kann vor der Feinreinigung eine Homologentrennung durchgeführt werden. Hier ist insbesondere die teilweise Abtrennung von MMDI aus dem von Lösungsmittel, Phosgen und Chlorwasserstoff weitest gehend befreiten Isocyanat-Gemisch enthaltend MMDI und PMDI unter Erhalt einer MMDI-Fraktion, die PMDI allenfalls in unbedeutenden Spuren enthält (Roh-MMDI), und eines Gemisches aus PMDI und MMDI, zu nennen.

Die Aufarbeitung eines Rohisocyanatstroms im großtechnischen Maßstab ist nicht trivial, weil viele verschiedene Anforderungen gleichzeitig zu berücksichtigen sind. Neben der Gewinnung des Zielprodukts in möglichst reiner Form sind hier die möglichst verlustfreie Rückgewinnung von Phosgen, Chlorwasserstoff und Lösungsmittel zu nennen, insbesondere zum Zwecke der Rezyklierung derselben (ggf. nach weiterer Umsetzung wie etwa Chlorwasserstoff zu Chlor) in den Prozess. Dies alles muss unter möglichst wirtschaftlichen Bedingungen, d. h. unter möglichst geringem Energieverbrauch und möglichst geringem Verlust an Wertprodukt (insbesondere an Isocyanat, das bei nicht optimal ausgestalteter Aufarbeitung unerwünschte Folgereaktionen eingehen kann) erfolgen.

Die Aufarbeitung eines Rohisocyanats, insbesondere von rohem MDI und speziell von Roh-MMDI, durch Destillation wurde schon vielfach beschrieben.

DE 3145010 A1 befasst sich mit einem Verfahren zur Herstellung von 4,4'-MMDI hoher Reinheit durch destillative Abtrennung von 4,4'-MMDI-Isomeren aus durch Phosgenierung von Anilin/Formaldehyd-Kondensaten gewonnenem MDI in einer Destillationsstufe (1), weitere Destillation des hierbei anfallenden Kopfprodukts in einer Destillationsstufe (2), wobei 0,5 bis 20 Gew.-% der in die Stufe (2) eingetragenen Produktmenge als Sumpf der Stufe (2) abgezogen werden, anschließende Abtrennung von 2,2'- und 2,4'-MMDI aus der als Kopfprodukt der Stufe (2) erhaltenen Fraktion in einer nachgeschalteten Destillationsstufe (3) und destillative Aufarbeitung des in der Stufe (3) anfallenden Sumpfprodukts unter Gewinnung von hochreinem 4,4'-MMDI. Dabei werden die Temperaturen der Kondensatorausgänge der Destillationsstufe (1), (2) und (3) dergestalt auf 130 °C bis 230 °C eingestellt, dass die Temperaturen 10°C bis 100°C unter der jeweils durch das Vakuum vorgegebenen Brüdentemperatur liegen. Ferner wird die Aufarbeitung des in der Stufe (3) anfallenden Sumpfes zweistufig dergestalt durchgeführt, dass in einer ersten Endstufe (4) 50 Gew.-% bis 90 Gew.-% des Sumpfes der Stufe (3) in Form von reinem 4,4'-MMDI als Kopfprodukt isoliert werden und der Sumpf der ersten Endstufe (4) in einer zweiten Endstufe (4') in einen weiteren Anteil an reinem 4,4'-MMDI als Kopfprodukt und einen Destillationsrückstand als Sumpf zerlegt wird.

EP 1 561 746 A2 beschreibt ein Verfahren zur Herstellung einer Fraktion von Diisocyanaten der Diphenylmethanreihe enthaltend mindestens 99 Gew.-% 2-Kern-Methylendiphenyldiisocyanat bezogen auf die Masse der Fraktion, bei dem man
a) Anilin und Formaldehyd in Gegenwart eines sauren Katalysators zu Di- und Polyaminen der Diphenylmethanreihe enthaltend 2-Kern-Methylendiphenyldiamin umsetzt, und
b) die Di- und Polyamine der Diphenylmethanreihe enthaltend 2-Kern-Methylendiphenyldiamin gegebenenfalls in Gegenwart eines Lösungsmittels phosgeniert, wobei man ein rohes Di- und Polyisocyanat erhält, und
c) aus dem rohen Di- und Polyisocyanat eine Fraktion enthaltend mindestens 95 Gew.-% 2-Kern-Methylendiphenyldiisocyanat mit einem Gehalt an 4,4'-MDI von 49 Gew.-% bis 95,99 Gew.-%, einem Gehalt an 2,4'-MDI von 4 Gew.-% bis 45 Gew.-% und einem Gehalt an 2,2'-MDI von 0,01 Gew.-% bis 20 Gew.-%, bezogen auf die Masse der Fraktion, abtrennt, und
d) gegebenenfalls aus der in Schritt c) erhaltenen Fraktion 4,4'-MDI zu 10 % bis 98 % entfernt, und
e) aus der in Schritt c) oder in Schritt d) erhaltenen Fraktion 2,2'-MDI ganz oder teilweise abtrennt, wobei man eine Fraktion enthaltend 0 Gew.-% bis 0,4 Gew.-% 2,2'-MDI, 1 Gew.-% bis 95 Gew.-% 4,4'-MDI und 5 Gew.-% bis 98,6 Gew.-% 2,4'-MDI, bezogen auf die Masse der MDI-Isomeren, erhält.

EP 1 686 112 A1 beschreibt ein Verfahren zur Herstellung von an 2,2'-MMDI armem 2,4'-MMDI durch Destillation eines Gemisches isomerer Diisocyanatodiphenylmethane, wenigstens bestehend aus 2,2'-MMDI, 2,4'-MMDI und 4,4'-MMDI unter Einsatz mindestens einer Trennwandkolonne. Es wird ein Gemisch erhalten, das 85 Gew.-% bis 99 Gew.-%, 2,4'-MMDI, maximal 15 Gew.-% 4,4'-MMDI und maximal 0,2 Gew.-% 2,2'-MMDI enthält.

EP 1 792 895 A1 beschreibt ein Verfahren zur Herstellung von 4,4'-MMDI, bei dem
a) Anilin und Formaldehyd im Molverhältnis 1,7 bis 4 : 1 in Gegenwart eines sauren Katalysators zu Di- und Polyaminen der Diphenylmethanreihe umgesetzt werden, und
b) die Di- und Polyamine mit Phosgen zu den entsprechenden Di- und Polyisocyanaten der Diphenylmethanreihe umgesetzt und ggf. destillativ getrennt werden, wobei ein Gemisch von Di- und Polyisocyanaten der Diphenylmethanreihe (MDI) enthaltend 44 Gew.-% bis 80 Gew.-% 4,4'-MMDI, sowie 1 Gew.-% bis 12 Gew.-% an 2,4'- und / oder 2,2'-MMDI in der Summe, und 10 Gew.-% bis 55 Gew.-% an PMDI, bezogen auf das Gewicht des MDI, erhalten wird, und
c) das MDI durch Destillation und / oder durch Kristallisation in genau zwei Fraktionen aufgetrennt wird, wobei die erste Fraktion in Mengen von 5 Gew.-% bis 40 Gew.-% der Menge des MDI erhalten wird und die zweite Fraktion in Mengen von 60 Gew.-% bis 95 Gew.-% der Menge des MDI erhalten wird, und wobei die erste Fraktion mindestens 97 Gew.-% an 4,4'-MMDI sowie maximal 3 Gew.-% an 2,4'-MMDI, bezogen auf das Gewicht der ersten Fraktion, enthält und die zweite Fraktion 30 Gew.-% bis 60 Gew.-% an 4,4'-MMDI, 1 Gew.-% bis 12 Gew.-% an 2,4'-MMDI und maximal 2 Gew.-% an 2,2'-MMDI sowie 35 Gew.-% bis 65 Gew.-% an PMDI, bezogen auf das Gewicht der zweiten Fraktion, enthält.

Obwohl nicht immer explizit erwähnt, fallen in allen Prozessen zur Herstellung von Isocyanaten Nebenkomponenten an, die leichter als das herzustellende Isocyanat sieden (sog. Leichtsieder; z. B. Phenylisocyanat im Fall der Herstellung von MDI) und im Aufarbeitungsprozess weitest möglich abgetrennt werden müssen. Leichtsieder werden meist über eine Abgasaufarbeitung einer Verbrennung zugeführt. Die Leichtsiederabtrennung birgt die Gefahr des Mittrisses von Wertprodukt. Hierauf geht der Stand der Stand der Technik nicht oder zumindest nicht hinreichend ein.

Als Alternative zu oder insbesondere auch in Verbindung mit einer destillativen Aufarbeitung können rohe Isocyanate auch durch Kristallisation (insbesondere Schmelzkristallisation und/oder Suspensionskristallisation) aufgearbeitet werden. Dies ist beispielsweise in WO2010/040675 A1 und WO2013/081873 A1 beschrieben. Wird eine Isocyanat-Fraktion durch Kristallisation gewonnen, so verbleiben Nebenkomponenten (wie die genannten leichter als das angestrebte Isocyanat siedenden Verbindungen) im Allgemeinen in der nicht kristallisierten Phase.

Unabhängig von der genauen Art der Aufarbeitung bestand daher ein Bedarf an weiteren Verbesserungen in der Aufarbeitung von rohem MDI, speziell von Roh-MMDI. Insbesondere wäre es wünschenswert, Nebenkomponenten, speziell die sog. Leichtsieder, vom angestrebten Zielprodukt unter möglichst geringem Verlust an Wertprodukt abzutrennen, ohne die Wirtschaftlichkeit des Verfahrens zu beeinträchtigen.

Diesem Bedarf Rechnung tragend ist ein Gegenstand der vorliegenden Erfindung ein **Verfahren zur Gewinnung von Methylen-Diphenylen-Diisocyanat und optional eines Gemisches aus Methylen-Diphenylen-Diisocyanat und Polymethylen-Polyphenylen-Polyisocyanat,** umfassend die Schritte:
α) Bereitstellen einer Methylen-Diphenylen-Diisocyanat und Nebenkomponenten enthaltenden Fraktion mit einem, auf ihre Gesamtmasse bezogenen, per Gaschromatographie bestimmten Massenanteil an Methylen-Diphenylen-Diisocyanat von mehr als 98,0 % (*"Roh-MMDI"*; in den Zeichnungen Strom 142),
   optional durch
   α.1) Abtrennung von Methylen-Diphenylen-Diisocyanat und Nebenkomponenten aus einer Methylen-Diphenylen-Diisocyanat und Polymethylen-Polyphenylen-Polyisocyanat enthaltenden Fraktion (*"Roh-MDI"*, in den Zeichnungen Strom 100) unter Erhalt
      (i) eines an Polymethylen-Polyphenylen-Polyisocyanat angereicherten Gemisches aus Methylen-Diphenylen-Diisocyanat und Polymethylen-Polyphenylen-Polyisocyanat (*"MDI"*, in den Zeichnungen Strom 141) und
      (ii) der Methylen-Diphenylen-Diisocyanat und Nebenkomponenten enthaltenden Fraktion mit einem, auf ihre Gesamtmasse bezogenen, per Gaschromatographie bestimmten Massenanteil an Methylen-Diphenylen-Diisocyanat von mehr als 98,0 % (142);
β) Reinigung, umfassend eine Isomerentrennung, der Methylen-Diphenylen-Diisocyanat und Nebenkomponenten enthaltenden Fraktion (142) durch Destillation und/oder Kristallisation in zwei oder mehr, bevorzugt in drei bis zehn, besonders bevorzugt in vier bis acht, Teilschritten (a, b, ...) unter Erhalt mindestens von
   (i) zwei oder mehr, bevorzugt zwei bis vier, besonders bevorzugt zwei bis drei, Methylen-Diphenylen-Diisocyanat-Reinfraktionen (140-1, 140-2, ...), die jeweils einen, auf ihre Gesamtmasse bezogenen, per Gaschromatographie bestimmten Massenanteil an Methylen-Diphenylen-Diisocyanat von 99,9 % oder mehr aufweisen und
   (ii) einer Nebenkomponenten-Fraktion (150) mit einem, auf ihre Gesamtmasse bezogenen, per Gaschromatographie bestimmten Massenanteil an Methylen-Diphenylen-Diisocyanat im Bereich von 20,0 % bis 98,0 %,
wobei die in Schritt β) erhaltene Nebenkomponenten-Fraktion (150)
in einen der Teilschritte (a, b,...) von Schritt β), *in dem keine der Methylen-Diphenylen-Diisocyanat-Reinfraktionen (140-1, 140-2, ...) aus Schritt β) als Destillat oder Kristallisat gewonnen wird,*
und/oder, insofern durchgeführt,
in Schritt α.1)
zurückgeführt wird.

Die *"Bereitstellung"* des Roh-MMDI in Schritt α) kann dabei sowohl durch Herstellung des Roh-MMDI in derselben Produktionsstätte, in welcher die Reinigung gemäß Schritt β) durchgeführt wird, als auch durch Herstellung des Roh-MMDI an anderer Stelle und Transport dieses Roh-MMDI zu der Produktionsstätte, in welcher die Reinigung gemäß Schritt β) durchgeführt wird, erfolgen. In letzterem Fall erfolgt die Bereitstellung gemäß Schritt α) einfach durch Entnahme des anderenorts hergestellten Roh-MMDI aus einem Transport- oder Lagertank oder auch aus einer Rohrfernleitung.

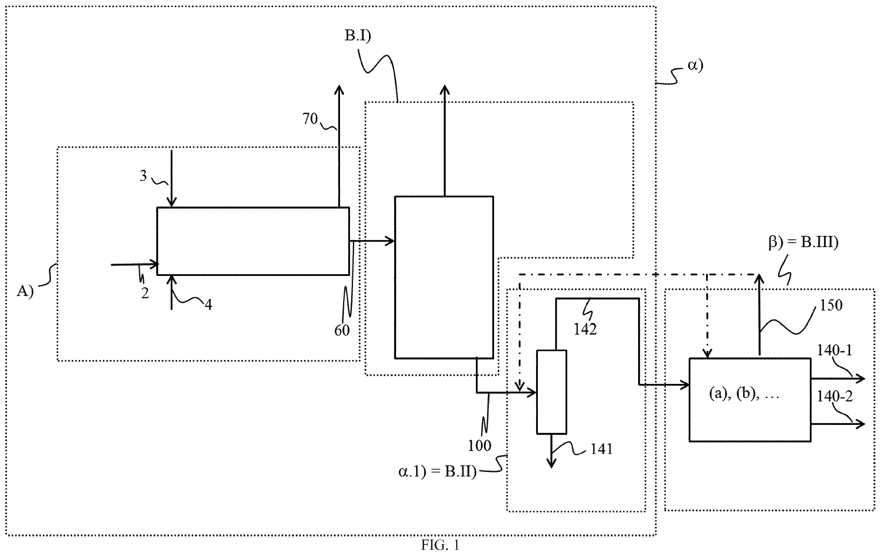

Besonders zweckmäßig ist jedoch ein integriertes Verfahren zur Herstellung und Reinigung von MDI und MMDI, *in welchem Schritt α) die Herstellung mindestens eines Teils, insbesondere sämtlichen, des in Schritt β) zu reinigenden Roh-MMDI* umfasst. Insbesondere ist daher ein weiterer Gegenstand der Erfindung ein **Verfahren zur Herstellung von Methylen-Diphenylen-Diisocyanat (1a) und eines Gemisches (1, MDI) aus Methylen-Diphenylen-Diisocyanat (1a) und Polymethylen-Polyphenylen-Polyisocyanat (1b)** aus einem Gemisch (2, MDA) aus Methylen-Diphenylen-Diamin (2a) und Polymethlyen-Polyphenylen-Polyamin (2b) umfassend die Schritte:
A) - *Bestandteil von Schritt α)* - Umsetzung von MDA (2) mit Phosgen (3) in Gegenwart eines organischen Lösungsmittels (4), wobei Phosgen (3) im stöchiometrischen Überschuss bezogen auf alle vorhandenen primären Aminogruppen eingesetzt wird, unter Erhalt eines flüssigen, Methylen-Diphenylen-Diisocyanat und Polymethylen-Polyphenylen-Polyisocyanat und Nebenkomponenten (sowie organisches Lösungsmittel, gelösten Chlorwasserstoff und gelöstes Phosgen) enthaltenden Stroms (60) und eines gasförmigen, Chlorwasserstoff und Phosgen enthaltenden Stroms (70);
B) Aufarbeitung mindestens des flüssigen, Methylen-Diphenylen-Diisocyanat und Polymethylen-Polyphenylen-Polyisocyanat und Nebenkomponenten enthaltenden Stroms (60), umfassend:
   B.I) - *Bestandteil von Schrittα)* - eine Vorreinigung unter Abtrennung eines ersten Teils der Nebenkomponenten unter Gewinnung einer flüssigen, an Nebenkomponenten (sowie an organischem Lösungsmittel, Chlorwasserstoff und Phosgen) abgereicherten, Methylen-Diphenylen-Diisocyanat und Polymethylen-Polyphenylen-Polyisocyanat enthaltenden Fraktion (100);
   B.II) - *Bestandteil von Schrittα), entsprechend Schritt α.1)* - Abtrennung von Methylen-Diphenylen-Diisocyanat und eines weiteren Teils der Nebenkomponenten aus der an Nebenkomponenten abgereicherten, Methylen-Diphenylen-Diisocyanat und Polymethylen-Polyphenylen-Polyisocyanat enthaltenden Fraktion (100) unter Erhalt
      (i) eines an Polymethylen-Polyphenylen-Polyisocyanat angereicherten Gemisches (in den Zeichnungen 141, entspricht 1) aus Methylen-Diphenylen-Diisocyanat und Polymethylen-Polyphenylen-Polyisocyanat und
      (ii) einer Methylen-Diphenylen-Diisocyanat und Nebenkomponenten enthaltenden Fraktion (142) mit einer, auf ihre Gesamtmasse bezogenen, per Gaschromatographie bestimmten Massenanteil an Methylen-Diphenylen-Diisocyanat von mehr als 98,0 %;
   B.III) - *entsprechend Schritt β)* - Reinigung, umfassend eine Isomerentrennung, der Methylen-Diphenylen-Diisocyanat und Nebenkomponenten enthaltenden Fraktion (142) durch Destillation und/oder Kristallisation in zwei oder mehr, bevorzugt in drei bis zehn, besonders bevorzugt in vier bis acht, Teilschritten (a, b,...) unter Erhalt mindestens von
      (i) zwei oder mehr, bevorzugt zwei bis vier, besonders bevorzugt zwei bis drei, Methylen-Diphenylen-Diisocyanat-Reinfraktionen (in den Zeichnungen 140-1, 140-2, ...; entspricht jeweils la), die jeweils einen, auf ihre Gesamtmasse bezogenen, per Gaschromatographie bestimmten Massenanteil an Methylen-Diphenylen-Diisocyanat von 99,9 % oder mehr aufweisen und
      (ii) einer Nebenkomponenten-Fraktion (150) mit einem, auf ihre Gesamtmasse bezogenen, per Gaschromatographie bestimmten Massenanteil an Methylen-Diphenylen-Diisocyanat im Bereich von 20,0 % bis 98,0 %,
wobei die in Schritt B.III) erhaltene Nebenkomponenten-Fraktion (150)
in einen der Teilschritte (a, b,...) von Schritt B.III), *in dem keine der Methylen-Diphenylen-Diisocyanat-Reinfraktionen (140-1, 140-2,* ...) *aus Schritt B.III) als Destillat oder Kristallisat gewonnen wird,*
und/oder
in Schritt B.II)
zurückgeführt wird (vgl. hierzu auch **FIG. 1****).**

In diesem **integrierten Verfahren zur Herstellung und Reinigung von MDI** und MMDI sind die Schritte A), B.I) und B.II) Bestandteil des Schrittes α), wobei Schritt B.II) dem Schritt α.1) entspricht. Ferner entspricht Schritt B.III) dem Schritt β).

Unter *"Nebenkomponenten"* werden erfindungsgemäß alle Bestandteile von Produktströmen und -fraktionen verstanden, die keinem herzustellenden Wertprodukt (MMDI, MDI) entsprechen und auch nicht organisches Lösungsmittel, Chlorwasserstoff oder Phosgen sind. Beispiele für typische Nebenkomponenten in diesem Sinne sind Phenylisocyanat (PHI) und Acridinhydrochlorid (welches sehr leicht sublimiert und dadurch leicht zum Bestandteil von Destillatfraktionen werden kann). Die in Schritt β) bzw. Schritt B.III) anfallende Nebenkomponenten-Fraktion (150) enthält solche Nebenkomponenten in einem Massenanteil von 2,0 % bis 80,0 %, bezogen auf die Gesamtmasse der Nebenkomponenten-Fraktion (150). Angesichts der Giftigkeit etwa von PHI (und angesichts seiner Monofunktionalität) sind solche Fraktionen selbst dann, wenn sich die Zusammensetzung am unteren Ende des genannten Konzentrationsbereiches für die Nebenkomponenten bewegt, nicht als verkaufsfähige Wertprodukte einsetzbar; sie werden daher *im Stand der Technik* üblicherweise verbrannt.

Unter einem "*Teilschritt (a, b, ...)"* in Schritt β) bzw. Schritt B.III) wird dabei ein Trennschritt verstanden, in welchem eine Ausgangsfraktion (insbesondere die Methylen-Diphenylen-Diisocyanat und Nebenkomponenten enthaltende Fraktion (142) oder eine daraus auf dem Weg zu den Endprodukten - den Methylen-Diphenylen-Diisocyanat-Reinfraktionen (140-1, 140-2, ...) - erhaltene Zwischen-Fraktion) in zwei oder mehr, insbesondere in zwei oder drei, Fraktionen aufgetrennt wird. Im Falle einer destillativen Reinigung umfasst ein Trennschritt insbesondere eine dem Fachmann geläufige Destillationskolonne (inklusive erforderlicher Peripheriegeräte wie Verdampfer, Kondensatoren und dergleichen). Im Falle einer Reinigung durch Kristallisation umfasst ein Trennschritt insbesondere einen dem Fachmann geläufigen Kristallisationsreaktor (auch "Kristaller" genannt).

Im Einklang mit dem in der Fachliteratur üblichen Sprachgebrauch bezeichnet ein *"Destillat"* im Sinne der vorliegenden Erfindungen eine durch partielle Verdampfung des flüssigen Zulaufs einer Destillation erhaltene Fraktion. Destillate in diesem Sinne werden einer Destillationskolonne am Kopf oder als Seitenabzug entnommen. Im Gegensatz zum Sumpfstrom einer Destillationskolonne, der im Sinne der vorliegenden Erfindung nicht als Destillat aufzufassen ist, bestehen Destillate - gegebenenfalls abgesehen von mitgerissenen Spuren nicht verdampfter Zulauf-Flüssigkeit - aus *in die Gasphase überführten Bestandteilen des flüssigen Zulaufs.* Destillate fallen zunächst gasförmig an und werden anschließend kondensiert. Eine solche Kondensation kann einerseits "gezielt" vorgenommen werden, indem das zunächst gasförmig anfallende Destillat einem Kondensator zugeführt wird. Eine "gezielte Kondensation" in diesem Sinne kann bereits *in* der Destillationskolonne selbst geschehen (etwa wenn *in* der Destillationskolonne im Bereich der oder oberhalb der Entnahmestelle des Destillats ein Kondensator angeordnet ist) oder nach gasförmiger Entnahme des Destillats in einem *außerhalb* der Destillationskolonne angeordneten Kondensator. Andererseits kann in einer Destillationskolonne, abhängig von deren genauer Ausgestaltung und Betriebsweise, aus einem gasförmig anfallenden Destillat auch bereits ohne Kontakt mit einem Kondensator ein flüssiger interner Rücklauf entstehen, der flüssig entnommen werden kann. Ein Destillat kann also einer Destillationskolonne je nach Ausgestaltung derselben und ihrer Betriebsweise *flüssig* oder *gasförmig* entnommen werden.

Im Einklang mit dem in der Fachliteratur üblichen Sprachgebrauch bezeichnet ein *"Kristallisat"* im Sinne der vorliegenden Erfindungen eine aus einem flüssigen Stoffgemisch auskristallisierte, also als Feststoff ausgefallene Verbindung. Die verbleibende Flüssigkeit wird als *Mutterlauge* bezeichnet.

Es folgt zunächst eine Kurzzusammenfassung verschiedener möglicher **Ausführungsformen der Erfindung,** wobei die Aufzählung an Ausführungsformen nicht als erschöpfend anzusehen ist:
In einer **ersten Ausführungsform der Erfindung,** die mit allen anderen Ausführungsformen kombiniert werden kann, ist Schritt α.1) umfasst.

In einer **zweiten Ausführungsform der Erfindung,** die eine besondere Ausgestaltung der ersten Ausführungsform ist, wird die Methylen-Diphenylen-Diisocyanat und Polymethylen-Polyphenylen-Polyisocyanat enthaltende Fraktion (100) durch folgende Schritte erhalten:
A) Umsetzung eines Gemisches (2) aus Methylen-Diphenylen-Diamin und Polymethylen-Polyphenylen-Polyamin mit Phosgen (3) in Gegenwart eines organischen Lösungsmittels (4), wobei Phosgen (3) im stöchiometrischen Überschuss bezogen auf alle vorhandenen primären Aminogruppen eingesetzt wird, unter Erhalt eines flüssigen, Methylen-Diphenylen-Diisocyanat und Polymethylen-Polyphenylen-Polyisocyanat und Nebenkomponenten enthaltenden Stroms (60) und eines gasförmigen, Chlorwasserstoff und Phosgen enthaltenden Stroms (70);
B) Aufarbeitung mindestens des flüssigen, Methylen-Diphenylen-Diisocyanat und Polymethylen-Polyphenylen-Polyisocyanat und Nebenkomponenten enthaltenden Stroms (60), umfassend:
   - eine Vorreinigung unter Abtrennung eines ersten Teils der Nebenkomponenten unter Gewinnung der flüssigen, an Nebenkomponenten abgereicherten, Methylen-Diphenylen-Diisocyanat und Polymethylen-Polyphenylen-Polyisocyanat enthaltenden Fraktion (100).

In einer **dritten Ausführungsform der Erfindung,** die eine besondere Ausgestaltung der zweiten Ausführungsform ist, wird das in Schritt A) eingesetzte organische Lösungsmittel (4) ausgewählt aus der Gruppe bestehend aus Monochlorbenzol, Dichlorbenzol, Dioxan, Toluol, Xylol, Methylenchlorid, Perchlorethylen, Trichlorfluormethan und Butylacetat.

In einer **vierten Ausführungsform der Erfindung,** die eine besondere Ausgestaltung der zweiten und dritten Ausführungsform ist, umfasst die Vorreinigung die folgenden Schritte:
(1) Abtrennung eines Gasstroms (90) enthaltend Chlorwasserstoff und Phosgen aus dem Methyl en-Diphenylen-Diisocyanat, Polymethylen-Polyphenylen-Polyisocyanat und Nebenkomponenten enthaltenden Strom (60);
(2) Abtrennung eines Gasstroms (110) enthaltend organisches Lösungsmittel (4) aus der in Schritt (1) nach Abtrennung des Gasstroms (90) enthaltend Chlorwasserstoff und Phosgen verbleibenden Flüssigphase, wobei die flüssige, an Nebenkomponenten abgereicherte, Methylen-Diphenylen-Diisocyanat und Polymethylen-Polyphenylen-Polyisocyanat enthaltende Fraktion (100) erhalten wird;
   und optional,
(3) Trennung des Gasstroms (110) enthaltend organisches Lösungsmittel (4) in einen flüssigen Strom (120) enthaltend organisches Lösungsmittel (4) und einen Gasstrom (130) enthaltend Phosgen.

In einer **fünften Ausführungsform der Erfindung,** die eine besondere Ausgestaltung der zweiten bis vierten Ausführungsform ist, wird in Schritt B) auch der gasförmige, Chlorwasserstoff und Phosgen enthaltende Strom (70) aufgearbeitet, wobei diese Aufarbeitung umfasst:
- Abtrennung von Phosgen aus dem gasförmigen, Chlorwasserstoff und Phosgen enthaltenden Strom (70), insbesondere nach Vereinigung mit dem Gasstrom (90) enthaltend Chlorwasserstoff und Phosgen, unter Erhalt eines Gasstroms (170) enthaltend Chlorwasserstoff, wobei, sofern vorhanden, auch der Gasstrom (130) enthaltend Phosgen diesem Phosgenabtrennungsschritt unterzogen wird;
   und optional den weiteren Schritt
- Abtrennung von Chlorwasserstoff aus dem Gasstrom (170) enthaltend Chlorwasserstoff.

In einer **sechsten Ausführungsform der Erfindung,** die eine besondere Ausgestaltung der zweiten bis fünften Ausführungsform ist, wird Schritt β) destillativ durchgeführt.

In einer **siebten Ausführungsform der Erfindung,** die eine besondere Ausgestaltung der sechsten Ausführungsform ist, umfasst Schritt β) vier bis acht Teilschritte, wobei jeder Teilschritt einer Destillation in einer Destillationskolonne ohne Trennwand entspricht, wobei die erste Methylen-Diphenylen-Diisocyanat-Reinfraktion (140-1) und die zweite Methylen-Diphenylen-Diisocyanat-Reinfraktion (140-2) in verschiedenen Destillationskolonnen jeweils als Destillat erhalten werden, wobei die Nebenkomponenten-Fraktion (150) in einer Destillationskolonne, die von denen zur Gewinnung der ersten und zweiten Methylen-Diphenylen-Diisocyanat-Reinfraktion verschieden ist, als Destillat gewonnen wird, wobei in dieser Destillationskolonne eine dritte Methylen-Diphenylen-Diisocyanat-Reinfraktion (140-3) als Sumpfprodukt anfällt.

In einer **achten Ausführungsform der Erfindung,** die eine besondere Ausgestaltung der siebten Ausführungsform ist, wird die Nebenkomponenten-Fraktion (150) in den Zulauf der Destillationskolonne, in welcher die Nebenkomponenten-Fraktion (150) erhalten wurde, eingespeist.

In einer **neunten Ausführungsform der Erfindung,** die eine andere besondere Ausgestaltung der sechsten Ausführungsform ist, umfasst Schritt β) zwei oder mehr Teilschritte, wovon mindestens ein Teilschritt in einer Trennwandkolonne durchgeführt wird.

In einer **zehnten Ausführungsform der Erfindung,** die eine besondere Ausgestaltung der neunten Ausführungsform ist, wird der in Schritt α.1) erhaltene Methylen-Diphenylen-Diisocyanat und Nebenkomponenten enthaltende Strom (142) in Schritt β) in eine Trennwandkolonne geleitet, welcher zwei vorgereinigte Methylen-Diphenylen-Diisocyanat-Fraktionen (140-11, 140-22) flüssig als Seitenströme entnommen werden, und welcher ein Nebenkomponenten und Methylen-Diphenylen-Diisocyanat enthaltender Kopfstrom entnommen wird,
wobei die vorgereinigten Methylen-Diphenylen-Diisocyanat-Fraktionen (140-11, 140-22, ...) in weiteren Destillationsstufen zu einer ersten und einer zweiten Methylen-Diphenylen-Diisocyanat-Reinfraktionen (140-1, 140-2) feingereinigt werden,
wobei der Nebenkomponenten und Methylen-Diphenylen-Diisocyanat enthaltende Kopfstrom der Trennwandkolonne in einer Destillationskolonne, die optional als Seitenabzugskolonne mit oder ohne Trennwand ausgestaltet sein kann, unter Erhalt der Nebenkomponenten-Fraktion (150) als Kopfstrom, einer dritten Methylen-Diphenylen-Diisocyanat-Reinfraktion (140-3) als Sumpfstrom und optional einer vierten Methylen-Diphenylen-Diisocyanat-Reinfraktion (140-4) als Seitenstrom destilliert wird,
wobei die Nebenkomponenten-Fraktion (150) in Schritt α.1) oder in die Trennwandkolonne aus Schritt β) zurückgeführt wird.

In einer **elften Ausführungsform der Erfindung,** die mit allen Ausführungsformen, in denen Schritt β) nicht rein destillativ durchgeführt wird, kombiniert werden kann, umfasst Schritt β) mindestens einen Teilschritt, in welchem eine Kristallisation durchgeführt wird, wobei das in der Kristallisation erhaltene Kristallisat eine Methylen-Diphenylen-Diisocyanat-Reinfraktion ist oder durch weitere Reinigung in eine Methylen-Diphenylen-Diisocyanat-Reinfraktion überführt werden kann.

In einer **zwölften Ausführungsform der Erfindung,** die eine besondere Ausgestaltung der elften Ausführungsform ist, wird die in dem mindestens einen Teilschritt, in welchem eine Kristallisation durchgeführt wird, anfallende Mutterlauge in mindestens zwei weiteren Teilschritten destilliert, wobei mindestens eine weitere Methylen-Diphenylen-Diisocyanat-Reinfraktion und die Nebenkomponenten-Fraktion (150) erhalten werden.

In einer **dreizehnten Ausführungsform der Erfindung,** die eine besondere Ausgestaltung der zwölften Ausführungsform ist, wird die Mutterlauge in drei weiteren Teilschritten destilliert, in welchen zwei Methylen-Diphenylen-Diisocyanat-Reinfraktionen erhalten werden.

Die zuvor kurz geschilderten Ausführungsformen und weitere mögliche Ausgestaltungen der Erfindung werden im Folgenden näher erläutert. Verschiedene Ausführungsformen sind, sofern sich aus dem Kontext für den Fachmann nicht eindeutig das Gegenteil ergibt, beliebig miteinander kombinierbar. Die Detailschilderung erfolgt dabei anhand des integrierten Verfahrens zur Herstellung und Reinigung von MDI, was jedoch nicht einschränkend zu verstehen ist.

**FIG. 1** zeigt das erfindungsgemäße integrierte Verfahren zur Herstellung und Reinigung von MDI in seiner breitesten Form *rein schematisch.* Die Abtrennung von organischem Lösungsmittel, Chlorwasserstoff, Phosgen und eines ersten Teils der Nebenkomponenten in Schritt B.I) ist schematisch durch den nach oben führenden Pfeil angedeutet. Die erfindungsgemäße Rückführung der Nebenkomponenten-Fraktion (150) *in einen der Teilschritte (a, b,* ...) *von Schritt B.III), in dem keine der Methylen-Diphenylen-Diisocyanat-Reinfraktionen (140-1, 140-2, ...) aus Schritt* B.III) *gewonnen wird, und*/*oder in Schritt B.II)* ist durch "Strich-Punkt"-Pfeile dargestellt. Dabei ist die Darstellung der letzteren Variante als Einspeisung in den Strom (100) nicht einschränkend zu verstehen; selbstverständlich kann diese Rückführung auch erfolgen, in dem die Nebenkomponenten-Fraktion (150) dem Schritt (B.II) *getrennt von Strom (100)* zugeführt wird.

Die kontinuierliche oder semi-kontinuierliche, bevorzugt kontinuierliche, Produktion von MDI aus MDA in **Schritt A)** kann gemäß einem aus dem Stand der Technik bekannten Verfahren erfolgen. Geeignete Verfahren sind beispielsweise in EP 2 077 150 A1, EP 1 616 857 A1, EP 1 873 142 A1, EP 0 716 079 A1 und EP 0 314 985 B1 beschrieben. Konzentrationen und Mengenströme der Edukte Amin (2) und Phosgen (3) werden dabei bevorzugt so gewählt, dass sich bei der Vermischung der Reaktionspartner ein molares Verhältnis von Phosgen zu primären Aminogruppen von 1,1 : 1 bis 30 : 1, besonders bevorzugt von 1,25 : 1 bis 3 : 1, einstellt.

Erfindungsgemäß wird in Schritt A) ein organisches Lösungsmittel (4) eingesetzt. Geeignete erfindungsgemäß einsetzbare organische Lösungsmittel (4) sind dabei unter den Reaktionsbedingungen inerte Lösungsmittel wie z. B. Monochlorbenzol, Dichlorbenzol, (insbesondere das ortho-Isomer), Dioxan, Toluol, Xylol, Methylenchlorid, Perchlorethylen, Trichlorfluormethan oder Butylacetat. Das inerte Lösungsmittel (4) ist bevorzugt im Wesentlichen frei von Isocyanat (Massenanteil < 100 ppm angestrebt) und im Wesentlichen frei von Phosgen (Massenanteil < 100 ppm angestrebt), worauf bei Einsatz von rezyklierten Strömen zu achten ist. Bevorzugt wird daher nach einem Verfahren, wie in EP 1 854 783 A2 beschrieben, gearbeitet. Die Lösungsmittel können einzeln oder als beliebige Gemische der beispielhaft genannten Lösungsmittel eingesetzt werden. Vorzugsweise werden Monochlorbenzol (MCB) oder *ortho*-Dichlorbenzol (ODB) eingesetzt. MCB ist ganz besonders bevorzugt.

In **Schritt B)** erfolgt die Aufarbeitung mindestens des flüssigen, Methylen-Diphenylen-Diisocyanat und Polymethylen-Polyphenylen-Polyisocyanat und Nebenkomponenten enthaltenden Stroms (60).

Die Vorreinigung des flüssigen, Methylen-Diphenylen-Diisocyanat und Polymethylen-Polyphenylen-Polyisocyanat und Nebenkomponenten (sowie organisches Lösungsmittel, gelösten Chlorwasserstoff und gelöstes Phosgen) enthaltenden Stroms (60) in Schritt B.I) umfasst dabei bevorzugt die folgenden Schritte:
(1) Abtrennung eines Gasstroms (90) enthaltend Chlorwasserstoff und Phosgen aus dem Methylen-Diphenylen-Diisocyanat, Polymethylen-Polyphenylen-Polyisocyanat und Nebenkomponenten (sowie organisches Lösungsmittel, gelösten Chlorwasserstoff und gelöstes Phosgen) enthaltenden Strom (60);
(2) Abtrennung eines Gasstroms (110) enthaltend organisches Lösungsmittel (4) aus der in Schritt (1) nach Abtrennung des Gasstroms (90) enthaltend Chlorwasserstoff und Phosgen verbleibenden Flüssigphase, wobei die flüssige, an Nebenkomponenten abgereicherte, Methyl en-Diphenylen-Diisocyanat und Polymethylen-Polyphenylen-Polyisocyanat enthaltende Fraktion (100) erhalten wird;
   und optional,
(3) Trennung des Gasstroms (110) enthaltend organisches Lösungsmittel (4) in einen flüssigen Strom (120) enthaltend organisches Lösungsmittel (4) und einen Gasstrom (130) enthaltend Phosgen.

In dieser Ausführungsform ist also der *flüssige, an Nebenkomponenten abgereicherte, Methylen-Diphenylen-Diisocyanat und Polymethylen-Polyphenylen-Polyisocyanat enthaltende Strom (100)* die nach Abtrennung des Gasstroms (110) enthaltend organisches Lösungsmittel (4) verbleibende Flüssigphase.

Die Abtrennung von Methylen-Diphenylen-Diisocyanat und eines weiteren Teils der Nebenkomponenten aus der an Nebenkomponenten abgereicherten, Methylen-Diphenylen-Diisocyanat und Polymethylen-Polyphenylen-Polyisocyanat enthaltenden Fraktion (100) in Schritt B.II) erfolgt durch Destillation, Kristallisation oder eine Kombination aus beiden, bevorzugt durch Destillation.

Die Reinigung, umfassend eine Isomerentrennung, der Methylen-Diphenylen-Diisocyanat und Nebenkomponenten enthaltenden Fraktion (142) in zwei oder mehr Teilschritten (a, b,...) in Schritt B.III) erfolgt ebenfalls durch Destillation, Kristallisation oder eine Kombination aus beiden, bevorzugt durch Destillation.

Bevorzugt wird in Schritt B) auch der gasförmige, Chlorwasserstoff und Phosgen enthaltende Strom (70) aufgearbeitet, wobei diese Aufarbeitung dann Folgendes umfasst:
B.IV) Abtrennung von Phosgen aus dem gasförmigen, Chlorwasserstoff und Phosgen enthaltenden Strom (70), insbesondere nach Vereinigung mit dem Gasstrom (90) enthaltend Chlorwasserstoff und Phosgen, unter Erhalt eines Gasstroms (170) enthaltend Chlorwasserstoff, wobei, sofern vorhanden, auch der Gasstrom (130) enthaltend Phosgen diesem Phosgenabtrennungsschritt unterzogen wird;
   und optional,
B.V) Abtrennung von Chlorwasserstoff aus dem Gasstrom (170) enthaltend Chlorwasserstoff.

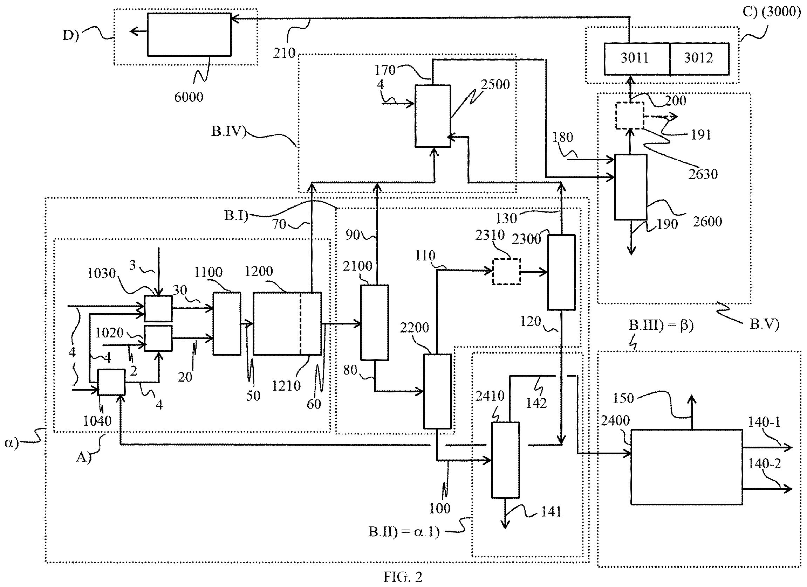

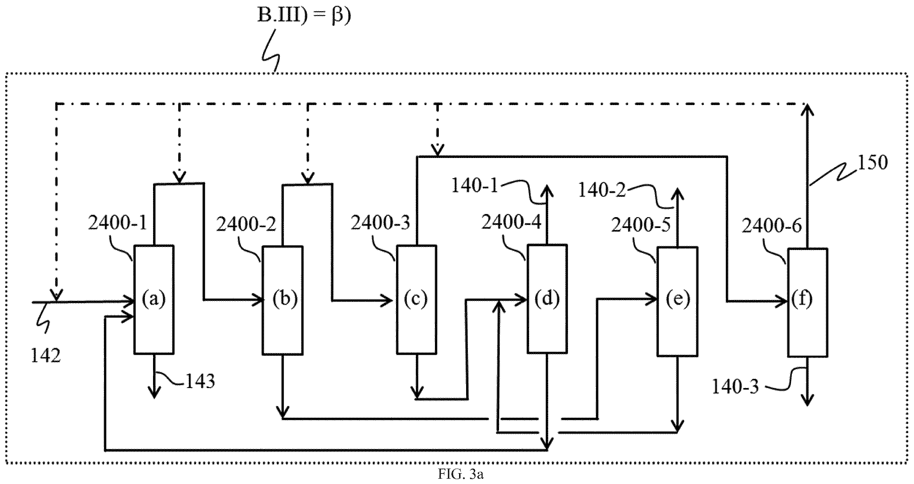

Eine mögliche Ausgestaltung des erfindungsgemäßen integrierten Verfahrens umfassend die Schritte A), B.I) (1),(2),(3), B.IV) und B.V) wird im Folgenden anhand der **FIG. 2** erläutert (die erfindungswesentliche Rückführung der Nebenkomponenten-Fraktion (150) ist dort aus Gründen der zeichnerischen Vereinfachung noch nicht gezeigt; hierzu sei auf die im Anschluss diskutierten Detailzeichnungen FIG. 3a-b verwiesen).

MDA (2) und Phosgen (3) werden in **Schritt A)** aus entsprechenden Vorratsbehältern (1020, 1030) einer geeigneten Mischzone (1100) zugeführt und dort vermischt. Dies geschieht in Form von Lösungen (20, 30) im Lösungsmittel (4). Geeignete Einrichtungen für die Ausgestaltung der Mischzone (1100) sind aus dem Stand der Technik hinreichend bekannt. Beispielhaft seien statische Mischorgane (vorzugsweise Düsen) und dynamische Mischorgane (vorzugsweise Rotor-Stator-Mischer) genannt. Nach der Vermischung wird das Reaktionsgemisch (50) in eine Reaktionszone (1200) geleitet. Diese ist eine Verweilzeiteinrichtung, in welcher das in der Mischzone (1100) erhaltene Gemisch ausreichende Gelegenheit zum Ausreagieren erhält. Geeignete Apparate sind aus dem Stand der Technik hinlänglich bekannt (z. B. von unten nach oben durchströmte, aufrecht angeordnete Rohrreaktoren, sog. "Phosgeniertürme"). Die Trennung des rohen Verfahrensproduktes in die Flüssigphase (60) und gasförmigen, Chlorwasserstoff und Phosgen enthaltenden Strom (70) erfolgt in der eigentlichen Reaktionszone selber oder in einer Abscheidezone (1210). Es ist auch möglich, die Mischzone und die Reaktionszone oder die Mischzone, die Reaktionszone und die Abscheidezone oder die Reaktionszone und die Abscheidezone in einen einzigen Apparat (z. B. in einen entsprechenden Reaktor) zu integrieren. Erfindungsgemäß können auch mehrere Mischzonen und/oder Reaktionszonen und/oder, sofern vorhanden, Abscheidezonen, seriell oder parallel geschaltet sein; z. B. in Form einer Kaskade mehrerer in Serie geschalteter Reaktoren. Das in der Reaktionszone (1200) erhaltene Verfahrensprodukt trennt sich in eine Flüssigphase (60), enthaltend neben MDI noch gelösten Chlorwasserstoff, überschüssiges gelöstes Phosgen, Lösungsmittel sowie Nebenkomponenten, und einen gasförmigen Strom (70), enthaltend Chlorwasserstoffgas, Phosgen und gasförmiges Lösungsmittel. An die Reaktionszone kann sich erforderlichenfalls eine Vorrichtung zur Spaltung von Carbaminsäurechlorid (nicht gezeigt in FIG. 2) anschließen. Die Flüssigphase (60) durchläuft in einem solchen Fall diese Vorrichtung, bevor sie der Aufarbeitung in Schritt B) unterworfen wird. Die dabei entstehende an Chlorwasserstoff angereicherte Gasphase wird bevorzugt mit dem gasförmigen Strom (70) vereint und gemeinsam weiter aufgearbeitet.

Die Aufarbeitung des rohen Isocyanats in **Schritt B)** umfasst zunächst in **Schritt B.1)** eine Abreicherung von Phosgen und Chlorwasserstoff aus der Flüssigphase (60) aus Schritt A) durch Auftrennung dieses flüssigen Stroms (60) in einen flüssigen, Lösungsmittel und Isocyanat enthaltenden Strom (80) und einen gasförmigen, Phosgen und Chlorwasserstoff enthaltenden Gasstrom (90) in einer Destillationsvorrichtung (2100; sog. "Entphosgenierkolonne"). Diese sog. Entphosgenierkolonne kann nach einem beliebigen aus dem Stand der Technik bekannten Verfahren betrieben werden, bevorzugt wie in EP 1 854 783 B1, insbesondere in den Abschnitten [0018] und [0023] beschrieben.

Der so erhaltene flüssige Strom (80) wird in einer Destillationsvorrichtung (2200; sog. "Lösungsmittelkolonne") in einen noch Lösungsmittel enthaltenden Gasstrom (110) und einen flüssigen, Isocyanat enthaltenden Strom (100) aufgetrennt. Dies kann nach einem beliebigen aus dem Stand der Technik bekannten Verfahren erfolgen, bevorzugt wie in EP 1 854 783 B1, insbesondere in den Abschnitten [0024] bis [0027], beschrieben. Die Destillationsvorrichtung (2200) kann auch zwei oder mehr Kolonnen umfassen (in FIG. 2 wird aus Gründen der zeichnerischen Vereinfachung diese Möglichkeit nicht gezeigt).

Der Prozessabgasstrom (110) wird, bevorzugt nach Verflüssigung in einem Kondensator (2310), in einer Destillationsvorrichtung (2300: sog. "Lösungsmittelstripper") in einen flüssigen, Lösungsmittel enthaltenden Strom (120) und einen Phosgen-haltigen Gasstrom (130) aufgetrennt. Dies kann nach einem beliebigen aus dem Stand der Technik bekannten Verfahren erfolgen, bevorzugt wie in EP 1 854 783 B1, insbesondere in den Abschnitten [0027] und [0028], beschrieben.

Die so erhaltenen Phosgen-haltigen Gasströme (70), (90) und (130) werden in einer Absorptionsvorrichtung (2500; sog. "Phosgenabsorber") durch Absorption in Lösungsmittel (4) unter Erhalt eines flüssigen, Lösungsmittel und Phosgen enthaltenden Stroms (160) und eines gasförmigen, Chlorwasserstoff und Lösungsmittel enthaltenden Prozessabgasstroms (170) gereinigt (d. h. von der Hauptmenge des Phosgens befreit), wobei bevorzugt die gasförmigen Phosgen-haltigen Prozessabgasströme (70) und (90) zunächst vereint werden und der vereinigte Phosgen-haltige Prozessabgasstrom aus (70) und (90) sowie der Phosgen-haltige Prozessabgasstrom (130) jeweils kondensiert und dann flüssig in die Absorptionsvorrichtung (2500) eingetragen werden **(Schritt B.IV)).** Diese Reinigung kann nach einem beliebigen aus dem Stand der Technik bekannten Verfahren erfolgen, bevorzugt wie in EP 2 093 215 A1 beschrieben.

Der auf diese Weise erhaltene Phosgen-haltige Gasstrom (170) enthält den in der Reaktion gebildeten Chlorwasserstoff und wird daher bevorzugt einer Chlorwasserstoffabtrennung in der Abtrenneinheit (2600) zugeführt **(Schritt B.V)).** Diese Abreicherung des Chlorwasserstoffgehalts erfolgt bevorzugt durch Absorption von Chlorwasserstoff in Wasser oder verdünnter Salzsäure als Absorptionsmittel (180) in einer weiteren Absorptionsvorrichtung (2600: "HCl-Absorptionskolonne"), wobei ein Salzsäure enthaltender Strom (190) und, bevorzugt nach Durchlaufen eines Brüdenkondensators (2630) zur weitest gehenden Abtrennung verflüssigbarer Bestandteile (191), ein gasförmiger, Lösungsmittel und ggf. gasförmige Nebenkomponenten enthaltender Phosgenhaltiger Prozessabgasstrom (200) erhalten wird. Dieser Schritt kann nach einem beliebigen aus dem Stand der Technik bekannten Verfahren erfolgen. Bevorzugt sind Verfahrensweisen wie in EP 1 743 882 B1, insbesondere in den Abschnitten [0027] bis [0038], beschrieben.

Als Absorptionsmittel (180) wird Wasser (z. B. Dampfkondensat) oder Salzsäure einer Konzentration im Bereich von 0,50 Massen-% bis 15,0 Massen-% (verdünnte Salzsäure) eingesetzt. Durch die bei der Absorption des Chlorwasserstoffs freiwerdende Wärme wird in Strom (170) enthaltendes Lösungsmittel überwiegend bis vollständig in den Gasstrom (200) überführt.

Der auf diese Weise erhaltene Phosgen-haltige Prozessabgasstrom (200) wird einer Phosgenzersetzung (Vorrichtung 3000, **C);** vorzugsweise umfassend zwei (oder mehr) parallel geschaltete, wechselseitig betriebene und regenerierte Phosgenzersetzungseinrichtungen (3011 und 3012) zugeführt. Hierbei wird Phosgen katalytisch, bevorzugt an Aktivkohle, unter Verwendung eines wässrigen Stroms zersetzt, wobei ein gasförmiger, gegebenenfalls Lösungsmittel und gegebenenfalls gasförmige Nebenkomponenten enthaltender Strom und ein salzsaurer flüssiger Strom erhalten werden. Bevorzugt werden das Prozessabgas und der wässrige Strom im Gleichstrom durch das Aktivkohlebett geführt. Der aus der Phosgenzersetzungseinrichtung austretende gasförmige Prozessabgasstrom (210) wird dann, optional nach Durchlaufen einer Adsorptionsvorrichtung zur Abtrennung letzter Lösungsmittelreste (nicht gezeigt in FIG. 2) einer Verbrennungseinrichtung (Vorrichtung 6000, **D))** zugeführt wird.

Zur **Reinigung** des rohen, von Lösungsmittel, Phosgen und Chlorwasserstoff weitgehend befreiten MDI (Roh-MDI 100) wird zunächst MMDI in der sog. "Polymerabtrennung" (Vorrichtung 2410) teilweise abgetrennt **(Schritt B.II)).** Diese Polymerabtrennung kann durch Destillation, Kristallisation oder eine Kombination aus beiden, bevorzugt durch Destillation, erfolgen. In jedem Fall fällt eine Fraktion (142) an, die neben MMDI einen weiteren Teil der im anfänglich angefallenen flüssigen Rohprodukt (60) enthaltenden Nebenkomponenten umfasst (Monomerfraktion, sog. Roh-MMDI). Schritt B.II) wird insbesondere so ausgestaltet, dass gegenüber MMDI niedriger siedende oder flüchtige Nebenkomponenten (wie beispielsweise PHI oder Reste an Lösungsmittel, die in den vorangegangen Schritten nicht vollständig abgetrennt wurden) so weit als möglich mit dieser Monomerfraktion abgetrennt werden, sodass das verbleibende Gemisch aus Methylen-Diphenylen-Diisocyanat und Polymethylen-Polyphenylen-Polyisocyanat (sog. Polymerfraktion, 141) bereits die für die weitere Verwendung angestrebte Reinheit aufweist. Dies gelingt am besten durch eine destillative Trennung. Es ist daher bevorzugt, das Roh-MDI (Strom 100) in einer Destillationskolonne (2410) in eine MMDI und Nebenkomponenten enthaltende Destillat-Fraktion (Strom 142) und in ein an PMDI angereichertes Gemisch aus MMDI und PMDI (Strom 141; Sumpfstrom) aufzutrennen. Mögliche Ausgestaltungen hierzu sind dem Fachmann bekannt und beispielsweise in EP 1 561 746 A2, insbesondere in Abschnitt [0038] und in den Beispielen, und in DE 3145010 A1, insbesondere auf Seite 7, Zeilen 17 ff. und in 1, beschrieben.

Erfindungsgemäß enthält das so abgetrennte Roh-MMDI (142) mehr als 98,00 Massen-% MMDI, bezogen auf seine Gesamtmasse. Das verbleibende MDI-Gemisch (141) aus MMDI und PMDI hat, also solches oder nach Zugabe von in Schritt B.III) anfallenden MMDI-haltigen Strömen, vielfältige Anwendungen in der Polyurethanchemie.

Die in **Schritt B.II)** erhaltene Monomerfraktion (142) kann - direkt oder nach zwischenzeitlicher Lagerung in einem Tankbehälter, der optional auch mit anderweitig bereitgestellten Monomerfraktionen, enthaltend Methylen-Diphenylen-Diisocyanat in einem auf ihre Gesamtmasse bezogenen, per Gaschromatographie bestimmten Massenanteil von mehr als 98,0 % sowie Nebenkomponenten, gespeist wird -, dem **Schritt B.III)** zugeführt werden.

Die Monomerfraktion (142) wird in **Schritt B.III)** in zwei oder mehr Teilschritten (a, b, ...) gereinigt, und zwar unter Erhalt mindestens von
(i) zwei oder mehr Methylen-Diphenylen-Diisocyanat-Reinfraktionen (140-1, 140-2, ...), die jeweils einen, auf ihre Gesamtmasse bezogenen, per Gaschromatographie bestimmten Massenanteil an Methylen-Diphenylen-Diisocyanat von 99,9 % oder mehr aufweisen und
(ii) einer Nebenkomponenten-Fraktion (150) mit einem, auf ihre Gesamtmasse bezogenen, per Gaschromatographie bestimmten Massenanteil an Methylen-Diphenylen-Diisocyanat im Bereich von 20,0 % bis 98,0 %.

Die Reinigung gemäß Schritt B.III) kann durch Destillation, Kristallisation oder eine Kombination aus beiden, bevorzugt durch Destillation, erfolgen. Aus Gründen der zeichnerischen Vereinfachung ist in der Übersichtszeichnung FIG. 2 dieser Schritt *nur rein schematisch* dargestellt. Das Bezugszeichen 2400 und das zugeordnete Symbol stehen hier summarisch für eine Mehrzahl von Aufarbeitungseinrichtungen. Mögliche Ausgestaltungen für die bevorzugte destillative Reinigung werden im Folgenden anhand der detaillierteren Abbildungen **FIG. 3a-b** gezeigt.

**FIG. 3a** zeigt eine mögliche Ausgestaltung von Schritt B.III) des erfindungsgemäßen Verfahrens unter Einsatz von sechs Destillationskolonnen (2400-1 bis 2400-6) ohne Trennwand. Peripheriegeräte wie z. B. Pumpen, Kondensatoren und Tanks sind aus Gründen der zeichnerischen Vereinfachung weggelassen. Die einzelnen (zunächst gasförmig anfallenden) Destillate werden jeweils bei einer Temperatur im Bereich von 50 °C bis 150 °C und einem Druck im Bereich von 5 mbar_{(abs.)} bis 15 mbar_{(abs.)} kondensiert. Die gewählte Temperatur hängt von der Zusammensetzung des Isomerengemisches des jeweiligen Destillates ab. Diejenigen Bestandteile der einzelnen Destillate, die unter den entsprechenden Druck- und Temperaturbedingungen nicht kondensieren, werden in das Vakuumsystem und schließlich in das Abgassystem abgeführt. Letztlich werden auf diese Weise auch die Leichtsieder (tiefer als 2,2'-MMDI siedende Komponenten) dem Destillationssystem entzogen; im Unterschied zum Stand der Technik jedoch unter deutlich geringeren Verlusten an Isocyanat.

Der MMDI und Nebenkomponenten enthaltende Strom (142) aus Schritt B.II) wird in einem ersten Teilschritt (a) in eine Destillationskolonne (2400-1) geleitet. In dieser Destillationskolonne werden vergleichsweise hoch siedende Nebenkomponenten und gegebenenfalls vorhandene mitgerissene PMDI-Anteile als Sumpfstrom (143) abgetrennt. Dieser Sumpfstrom enthält daneben noch Monomer-Anteile, insbesondere Anteile des am höchsten siedenden Isomers 4,4'-MMDI. Der Strom (143) weist einen, auf seine Gesamtmasse bezogenen, per Gaschromatographie bestimmten Massenanteil an Methylen-Diphenylen-Diisocyanat von weniger als 99,9 % (nämlich insbesondere von 50,0 % bis 99,5 %) auf und kann beispielsweise mit in Schritt B.II) erhaltenen MDI-Fraktionen (141) verschnitten werden.

Das in der ersten Destillationskolonne erhaltene Destillat wird nun in einem zweiten Teilschritt (b) in eine weitere Destillationskolonne (2400-2) geführt. Das Sumpfprodukt dieser Destillationskolonne enthält überwiegend das am höchsten siedende Isomer 4,4'-MMDI neben geringen Anteilen an 2,4'-MMDI, während das Destillat (Kopfprodukt) ein Gemisch aus allen Isomeren ist. Zur Erhöhung der Betriebssicherheit kann es sinnvoll sein, für diesen Teilschritt (b) zwei parallel geschaltete, wechselseitig betreibbare Destillationskolonnen vorzuhalten (nicht gezeigt in FIG. 3a).

Das Destillat der Destillationskolonne (2400-2) wird nun in einem dritten Teilschritt (c) in eine weitere Destillationskolonne (2400-3) geleitet, in welcher leicht siedende Nebenkomponenten zusammen mit Anteilen der MMDI-Isomeren als Destillat abgetrennt werden.

Das Sumpfprodukt dieser dritten Destillationskolonne (2400-3) wird in einem vierten Teilschritt (d) in eine weitere Destillationskolonne (2400-4) geführt, in welcher die erste MMDI-Reinfraktion (140-1), enthaltend überwiegend die Isomere 4,4'-MMDI und 2,4'-MMDI neben untergeordneten Anteilen an 2,2'-MMDI, als Destillat gewonnen wird. Diese MMDI-Reinfraktion (140-1) enthält bevorzugt 0,0 Massen-% bis 2,0 Massen-% 2,2'-MMDI, 30,0 Massen-% bis 70,0 Massen-% 2,4-MMDI und 30,0 Massen-% bis 70,0 Massen-% 4,4'-MMDI, bezogen auf die Gesamtmasse aller MMDI-Isomeren.

Das Sumpfprodukt der zweiten Destillationskolonne (2400-2) wird in einem fünften Teilschritt (e) in eine Destillationskolonne (2400-5) geleitet. Dort wird die zweite MMDI-Reinfraktion (140-2), enthaltend überwiegend das Isomer 4,4'-MMDI, als Destillat gewonnen. Es ist auch denkbar, anstelle einer einzigen Destillationskolonne (2400-5) zwei in Serie geschaltete Destillationskolonnen (2400-51, 2400-52) einzusetzen, wobei dann die zweite MMDI-Reinfraktion (140-2) als Destillat der in Strömungsrichtung zweiten Destillationskolonne (2400-52) erhalten wird (nicht gezeigt in FIG. 3a). In dieser Ausführungsform sind die in den Destillationskolonnen 2400-51 und 2400-52 jeweils durchgeführten Stofftrennungen *als eigene Teilschritte im Sinne der Erfindung aufzufassen.* In jedem Fall enthält die MMDI-Reinfraktion (140-2) bevorzugt 0,0 Massen-% bis 1,0 Massen-% 2,2'-MMDI, 0,1 Massen-% bis 5,0 Massen-% 2,4-MMDI und 94,0 Massen-% bis 99,9 Massen-% 4,4'-MMDI, bezogen auf die Gesamtmasse aller MMDI-Isomeren.

Das Sumpfprodukt dieser fünften Destillationskolonne (2400-5) wird gemeinsam mit dem Sumpfprodukt der dritten Destillationskolonne (2400-3) in die vierte Destillationskolonne (2400-4) eingespeist.

Das Destillat der dritten Destillationskolonne (2400-3) wird in einem sechsten Teilschritt (f) in eine weitere Destillationskolonne (2400-6, sog. "Leichtsiederkolonne") geführt. Dort wird die Nebenkomponenten-Fraktion (150) als Destillat abgetrennt, während als Sumpfprodukt eine dritte MMDI-Reinfraktion (140-3) enthaltend alle Isomeren anfällt. Diese kann entweder als solche weiterverwendet oder mit anderen Fraktionen, z. B. mit in Schritt B.II) erhaltenen MDI-Fraktionen (140), verschnitten werden. Diese MMDI-Reinfraktion (140-3) enthält bevorzugt 10,0 Massen-% bis 60,0 Massen-% 2,2'-MMDI, 30,0 Massen-% bis 80,0 Massen-% 2,4-MMDI und 0,0 Massen-% bis 20,0 Massen-% 4,4'-MMDI, bezogen auf die Gesamtmasse aller MMDI-Isomeren. Die Nebenkomponenten-Fraktion (150) weist bevorzugt einen, auf ihre Gesamtmasse bezogenen, Massenanteil an Methylen-Diphenylen-Diisocyanat im Bereich von 20,0 % bis 98,0 %, bevorzugt 60 % bis 98 %, auf.

Erfindungswesentlich ist nun, dass die Nebenkomponenten-Fraktion (150) an eine Stelle der Aufarbeitung zurückgeführt wird, in der *keine der Methylen-Diphenylen-Diisocyanat-Reinfraktionen (140-1, 140-2,* ...) *aus Schritt B.III) als Destillat gewonnen wird.* FIG. 3a zeigt hierzu die für diese Ausführungsform möglichen Durchführungsvarianten (dargestellt durch "Strich-Punkt"-Pfeile): Die Nebenkomponenten-Fraktion (150) kann - nach weitest gehender Verflüssigung; siehe oben - in den Zulauf der ersten Destillationskolonne (2400-1) und/oder in den Zulauf der zweiten Destillationskolonne (2400-2) und/oder in den Zulauf der dritten Destillationskolonne (2400-3) und/oder in den Zulauf der sechsten Destillationskolonne (2400-6) geführt werden. Aus energetischen Gründen ist eine Einspeisung nur in den Zulauf der sechsten Destillationskolonne (2400-6) bevorzugt.

Vollkommen überraschend wurde gefunden, dass diese Vorgehensweise nicht zu einer unakzeptablen Anreicherung von Leichtsiedern in den Wertproduktströmen führt, sondern die Leichtsieder in ausreichender Weise über das Abgassystem ausgetragen (und letztlich verbrannt) werden. Das erfindungsgemäße Verfahren erlaubt es somit, die in der Nebenkomponenten-Fraktion (150) enthaltenen MMDI-Anteile (die immerhin bis zu 98,0 Massen-% dieses Stroms ausmachen können), teilweise bis vollständig zu verwerten, ohne die Produktqualität nachteilig zu beeinflussen.

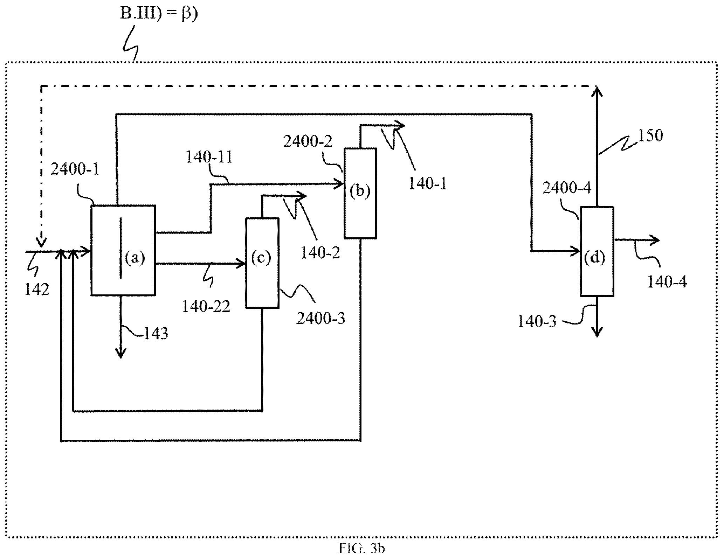

Abweichend von der Ausführungsform gemäß FIG. 3a ist es auch möglich, den Schritt B.III) unter Einsatz von Trennwandkolonnen durchzuführen und dadurch die Anzahl der erforderlichen Destillationskolonnen zu verringern. Dies ist in einer Ausführungsform, die mit nur vier Destillationskolonnen (2400-1 bis 2400-4) auskommt, in **FIG. 3b** gezeigt. Peripheriegeräte wie z. B. Pumpen, Kondensatoren und Tanks sind aus Gründen der zeichnerischen Vereinfachung weggelassen. Die einzelnen zunächst gasförmig anfallenden Destillate werden, in der jeweiligen Destillationskolonne oder in einem außerhalb derselben angeordneten Kondensator, jeweils bei einer Temperatur im Bereich von 50 °C bis 150 °C und einem Druck im Bereich von 5 mbar_{(abs.)} bis 15 mbar_{(abs.)} kondensiert. Die gewählte Temperatur hängt von der Zusammensetzung des Isomerengemisches des jeweiligen Destillates ab. Diejenigen Bestandteile der einzelnen Destillate, die unter den entsprechenden Druck- und Temperaturbedingungen nicht kondensieren, werden in das Vakuumsystem und schließlich in das Abgassystem abgeführt.

Der MMDI und Nebenkomponenten enthaltende Strom (142) aus Schritt B.II) wird in einem ersten Teilschritt (a) in eine Destillationskolonne (2400-1) mit Trennwand geleitet (sog. Trennwandkolonne). Dieser Trennwandkolonne werden zwei Seitenströme flüssig entnommen:
Der obere Seitenstrom (140-11) enthält ein Gemisch aus allen Isomeren und wird in einem zweiten Teilschritt (b) in einer zweiten Destillationskolonne (2400-2) - ohne Trennwand - feingereinigt, wobei als Destillat die MMDI-Reinfraktion (140-1) erhalten wird. Die MMDI-Reinfraktion (140-1) enthält bevorzugt 0,0 Massen-% bis 2,0 Massen-% 2,2'-MMDI, 30,0 Massen-% bis 70,0 Massen-% 2,4-MMDI und 30,0 Massen-% bis 70,0 Massen-% 4,4'-MMDI, bezogen auf die Gesamtmasse aller MMDI-Isomeren.

Der untere Seitenstrom (140-22) enthält überwiegend das am höchsten siedende Isomer 4,4'-MMDI neben geringen Anteilen an 2,4'-MMDI und wird einem dritten Teilschritt (c) in einer dritten Destillationskolonne (2400-3) - ohne Trennwand - feingereinigt, wobei als Destillat die MMDI-Reinfraktion (140-2) erhalten wird. Die MMDI-Reinfraktion (140-2) enthält bevorzugt 0,0 Massen-% bis 1,0 Massen-% 2,2'-MMDI, 0,1 Massen-% bis 5,0 Massen-% 2,4-MMDI und 94,0 Massen-% bis 99,9 Massen-% 4,4'-MMDI, bezogen auf die Gesamtmasse aller MMDI-Isomeren.

Die in den Teilschritten (b) und (c) anfallenden Sumpfströme werden in den Zulauf der Trennwandkolonne geführt.

Der in der Trennwandkolonne anfallende Sumpfstrom (143) enthält vergleichsweise hoch siedende Nebenkomponenten und gegebenenfalls vorhandene mitgerissene PMDI-Anteile. Dieser Sumpfstrom enthält daneben noch Monomer-Anteile, insbesondere Anteile des am höchsten siedenden Isomers 4,4'-MMDI. Der Strom (143) weist einen, auf seine Gesamtmasse bezogenen, per Gaschromatographie bestimmten Massenanteil an Methylen-Diphenylen-Diisocyanat von weniger als 99,9 % (nämlich insbesondere von 50,0 % bis 99,5 %) auf und kann beispielsweise mit in Schritt B.II) erhaltenen MDI-Fraktionen (141) verschnitten werden.

Der in der Trennwandkolonne anfallende Kopfstrom enthält die leichtsiedenden Nebenkomponenten neben Anteilen an MMDI. Dieser Strom wird in einem vierten Teilschritt (d) in einer Destillationskolonne (2400-4), die als Seitenabzugskolonne (in 3b ohne Trennwand; der Einsatz einer Trennwandkolonne an dieser Stelle ist jedoch auch möglich) ausgestaltet ist, von den Nebenkomponenten gereinigt, welche als Nebenkomponenten-Fraktion (150) über Kopf abgezogen werden, während als Sumpfprodukt eine dritte MMDI-Reinfraktion (140-3) enthaltend alle Isomeren anfällt. Diese kann entweder als solche weiterverwendet oder mit anderen Fraktionen, z. B. mit in Schritt B.II) erhaltenen MDI-Fraktionen (140), verschnitten werden. Diese MMDI-Reinfraktion (140-3) enthält bevorzugt 10,0 Massen-% bis 60,0 Massen-% 2,2'-MMDI, 30,0 Massen-% bis 80,0 Massen-% 2,4-MMDI und 0,0 Massen-% bis 20,0 Massen-% 4,4'-MMDI, bezogen auf die Gesamtmasse aller MMDI-Isomeren. Der Seitenabzugsstrom (140-4) weist üblicherweise eine Isomerenverteilung auf, die ebenfalls in diesem Bereich liegt, enthält aber im Allgemeinen mehr Nebenkomponenten. Die Anforderungen an eine MMDI-Reinfraktion (maximal 0,1 % Nebenkomponenten) werden jedoch erfüllt. Grundsätzlich ist es auch vorstellbar, die Destillationskolonne (2400-4) ohne Seitenabzug zu betreiben; je nach Betriebsweise gelangen dann die im Fall der Ausführung gemäß FIG. 3b im Seitenabzugsstrom enthaltenen MMDI-Bestandteile und Nebenkomponenten in die über Kopf abgezogene Nebenkomponenten-Fraktion (150) oder in den Sumpfstrom (140-3).

Die Nebenkomponenten-Fraktion (150) wird - nach weitest gehender Verflüssigung; siehe oben - in dieser Ausführungsform in den Zulauf der Trennwandkolonne (2400-1) geführt.

In allen zuvor geschilderten Ausführungsformen ist es auch möglich, die Nebenkomponenten-Fraktion (150) in Schritt B.II) (Polymerabtrennung) zurückzuführen. Hierzu kann die Nebenkomponenten-Fraktion (150) - nach weitest gehender Verflüssigung; siehe oben - in den Zulauf der Destillationskolonne (2410) geführt werden.

Der in den zuvor geschilderten Ausführungsformen anfallende Sumpfstrom (140-3) kann, erforderlichenfalls nach Abmischung mit anderen MMDI-Reinfraktionen, in vielen Bereichen Anwendung finden. Insbesondere sei an dieser Stelle die Verwendung in der Herstellung von Sportböden, Weichschäumen und Lebensmittelverpackungen sowie die Anwendung als Holzbindemittel erwähnt.

Neben den zuvor geschilderten Ausführungsformen mit rein destillativer Ausgestaltung des Schrittes B.III) kann dieser auch Kristallisationsschritte umfassen. Beispiele derartiger Ausführungsformen sind z. B. in EP1561746 A2 und dort zitierten Schriften, WO2010/095927 A1 sowie von M. Stepanski und P. Faessler in SULZER TECHNICAL REVIEW 4/2002, Seiten 14 bis 16, zu finden.

Bevorzugt sind *Kombinationen* aus Kristallisations- und Destillationsschritten. So kann beispielsweise in der Ausführungsform gemäß FIG. 3a die Trennaufgabe der zweiten Destillationskolonne (2400-2) durch einen Kristallisationsschritt ersetzt werden, in welchem das am höchsten siedende Isomer 4,4'-MMDI neben geringen Anteilen an 2,4'-MMDI auskristallisiert, während ein Gemisch aus allen Isomeren als Mutterlauge verbleibt. Die Mutterlauge kann anschließend in mindestens zwei weiteren Teilschritten unter Erhalt mindestens einer Methylen-Diphenylen-Diisocyanat-Reinfraktion destillativ aufgearbeitet werden. Bevorzugt geschieht dies in drei Teilschritten in den Destillationskolonnen 2400-3, 2400-4 und 2400-6 in der oben beschriebenen Weise, wobei zwei Methylen-Diphenylen-Diisocyanat-Reinfraktionen (140-1 und 140-3) erhalten werden. Das hoch 4,4'-MMDI-haltige Kristallisat kann zur weiteren Reinigung unter Erhalt der MMDI-Reinfraktion (140-2) ein zweites Mal kristallisiert oder - nach Verflüssigung - in der Destillationskolonne 2400-5 destilliert werden. Diese weitere Reinigung ist jedoch nicht zwingend erforderlich; wenn die die Destillation in der Destillationskolonne 2400-2 ersetzende Kristallisation die MMDI-Reinfraktion (140-2) bereits in ausreichender Reinheit liefert, ist eine weitere Kristallisation oder die Destillation in der Destillationskolonne 2400-5 verzichtbar.

Erfindungswesentlich ist auch bei dieser Kristallisationsschritte umfassenden Ausführungsform, dass die Nebenkomponenten-Fraktion (150) an eine Stelle der Aufarbeitung zurückgeführt wird, in der *keine der Methylen-Diphenylen-Diisocyanat-Reinfraktionen (140-1, 140-2,* ...) *aus Schritt B.III) als Destillat bzw. als Kristallisat gewonnen wird.* Dies bedeutet, dass bei Ersatz des Teilschrittes (b) durch eine solche Kristallisation, die die angestrebte MMDI-Reinfraktion (140-2) bereits in ausreichender Reinheit liefert, sodass weitere Destillations- oder Kristallisationsschritte entfallen, *die Nebenkomponenten-Fraktion (150) nicht in Teilschritt (b) zurückgeführt wird.*

### Beispiele:

### Analysenmethoden:

Viskosität: Bestimmung mittels Kugelfallviskosimeter oder Brookfield-Viskosimeter (Rotationsviskosimeter).
NCO-Wert: Umsetzung mit Dibutylamin und Rücktitration des nicht umgesetzten Dibutylamins mit einer HCl-Maßlösung.
Zusammensetzung: Gaschromatographie.

In den Beispielen 1 bis 4 wurden die **Schritte A) und B.I),** wie in WO2017/050776 A1, Seite 35, Zeile 2 bis Seite 36, Zeile 10, beschrieben, durchgeführt (wobei die Mengen an umgesetztem MDA unterschiedlich sein können). In Beispiel 5 wurden entsprechende Bedingungen einer Prozess-Simulation zugrunde gelegt.

Das auf diese Weise als Sumpfprodukt erhaltene Roh-MDI wurde in den Beispielen 1 bis 3 wie folgt einer **"Polymerabtrennung" gemäß Schritt B.II)** unterzogen (siehe auch FIG. 2):
Das Roh-MDI (entsprechend Strom 100 in 2, mit einem Mengenstrom von 5,4 t/h) wurde in einer Destillationskolonne (2410) in eine MMDI und Nebenkomponenten (die in den vorhergehenden Schritten durchgeführte Abtrennung von Nebenkomponenten wie Phenylisocyanat und Lösungsmittel gelingt nicht zu 100 %) enthaltende Fraktion (*Roh-MMDI*; Strom 142, 1,8 t/h) und in ein an PMDI angereichertes Gemisch aus MMDI und PMDI (*MDI*; Strom 141, 3,6 t/h) aufgetrennt. Die Destillationskolonne (2410) wurde bei einem Druck von 8 mbar_{(abs.)} und einer Sumpftemperatur von 220 °C betrieben.

In Beispiel 4 wurde **Schritt B.II)** in einer Seitenabzugskolonne (ohne Trennwand; Zulauf 63 t/h) bei einem Druck von 10 mbar_{(abs.)} und einer Sumpftemperatur von 225 °C durchgeführt, wobei das Roh-MMDI (142) als Seitenstrom entnommen wurde. In Beispiel 5 wurden entsprechende Bedingungen einer Prozess-Simulation zugrunde gelegt.

Die in den Beispielen 1 bis 4 jeweils so gewonnene MMDI und Nebenkomponenten enthaltende Fraktion (142) wurde in einen Lagertank (nicht gezeigt in 2) gepumpt. Aus diesem Tank wurde die Fraktion (142) als Ausgangsmaterial für die folgenden Beispiele entnommen.

### Beispiel 1 (Vergleichsbeispiel - Destillationskolonnen ohne Trennwand):

Mit Ausnahme der Rückführung der Nebenkomponenten-Fraktion (150) wurde die Reinigung und Isomerentrennung nach einem Verfahren gemäß 3a durchgeführt (die Nebenkomponenten-Fraktion (150) wurde verbrannt). Dabei wurden folgende Betriebsparameter eingehalten:

**Tabelle 1: Betriebsparameter in Beispiel 1**

| **Destillationskolonne** | **Kopfdruck** / **mbar_{(abs.)}** | **Sumpftemperatur** / **°C** | **Kopftemperatur** / **°C** | **Zulaufmenge** / **t h⁻¹** |
|---|---|---|---|---|
| 2400-1 | 7,0 | 205 | 196 | 9,80 *(Fraktion 142)* |
| | | | | 1,00 *(Sumpf aus 2400-4)* |
| 2400-2 | 6,0 | 212 | 190 | 9,80 *(Destillat aus 2400-1)* |
| 2400-3 | 6,0 | 210 | 188 | 1,95 *(Destillat aus 2400-2)* |
| 2400-4 | 6,0 | 213 | 201 | 1,80 *(Sumpf aus 2400-3)* |
| | | | | 1,00 *(Sumpf aus 2400-5)* |
| 2400-5 | 6,0 | 210 | 197 | *7,85 (Sumpf aus 2400-2)* |
| 2400-6 | 6,0 | 210 | 161 | 0,15 *(Destillat aus 2400-3)* |

Die folgenden Ströme verließen die Destillationssequenz:

**Tabelle 2: In Beispiel 1 erhaltene Produktströme**

| **Strom** | **Menge** / **t h⁻¹** | **Analytik** | **Anmerkung** |
|---|---|---|---|
| 143 | 1,00 | 26,6 Massen-% NCO Viskosität 217 mPa · s | Wird mit Strom 141 aus B.II) oder mit anderen Isocyanaten vermischt. |
| 140-1 | 1,80 | 44,9 % 4,4'-MMDI / 55,0 % 2,4'-MMDI / 0,1 % 2,2'-MMDI / | *Massenprozent bezogen auf Gesamtmasse MMDI, Nicht-MMDI-Bestandteile allenfalls in Spuren (< 10 ppm) vorhanden*; verkaufsfähiges Produkt. |
| 140-2 | 6,85 | 98,5 % 4,4'-MMDI / 1,5 % 2,4'-MMDI | *Massenprozent bezogen auf Gesamtmasse MMDI, Nicht-MMDI-Bestandteile allenfalls in Spuren (< 10 ppm) vorhanden*; verkaufsfähiges Produkt. |
| 140-3 | 0,10 | 6,3 % 4,4'-MMDI / 55,0 % 2,4'-MMDI / 38,7 % 2,2'-MMDI / | *Massenprozent bezogen auf Gesamtmasse MMDI, Nicht-MMDI-Bestandteile allenfalls in Spuren vorhanden (< 100 ppm);* grundsätzlich verkaufsfähiges Produkt; wird i. A. in den Zulauf der Polymerabtrennung aus B.II) eingespeist. |
| 150 | 0,050 | 97,5 % MMDI / 2,5 % Nebenkomponenten (PHI, MCB, etc.) | Wird der Verbrennung zugeführt. |

### Beispiel 2 (erfindungsgemäß - Destillationskolonnen ohne Trennwand)

Es wurde verfahren wie in Beispiel 1 mit dem Unterschied, dass die Nebenkomponenten-Fraktion (150) *als Bestandteil des Zulaufs der Destillationskolonne 2400-1* verwendet wurde. Dabei wurden folgende Betriebsparameter eingehalten:

**Tabelle 3: Betriebsparameter in Beispiel 2**

| **Destillationskolonne** | **Kopfdruck / mbar_{(abs.)}** | **Sumpftemperatur / °C** | **Kopftemperatur / °C** | **Zulaufmenge / t h⁻¹** |
|---|---|---|---|---|
| 2400-1 | 7,0 | 205 | 196 | 9,80 *(Fraktion 142)* |
| | | | | 1,00 *(Sumpf aus 2400-4)* |
| | | | | **0,035 *(Fraktion 150)*** |
| 2400- 2 | 6,0 | 212 | 190 | **9,84** *(Destillat aus 2400-1)* |
| 2400-3 | 6,0 | 210 | 188 | **1,99** *(Destillat aus 2400-2)* |
| 2400-4 | 6,0 | 213 | 201 | 1,80 *(Sumpf aus 2400-3)* |
| | | | | 1,00 *(Sumpf aus 2400-5)* |
| 2400-5 | 6,0 | 210 | 197 | 7,85 *(Sumpf aus 2400-2)* |
| 2400-6 | 6,0 | 210 | 161 | 0,15 *(Destillat aus 2400-3)* |

Die folgenden Ströme verließen die Destillationssequenz:

**Tabelle 4: In Beispiel 2 erhaltene Produktströme**

| **Strom** | **Menge / t h⁻¹** | **Analytik** | **Anmerkung** |
|---|---|---|---|
| 143 | 1,00 | **26,0** Massen-% NCO Viskosität **298** mPa · s | Wird mit Strom 141 aus B.II) oder mit anderen Isocyanaten vermischt. |
| 140-1 | 1,80 | 44,9 % 4,4'-MMDI / 55,0 % 2,4'-MMDI / 0,1 % 2,2'-MMDI / | *Massenprozent bezogen auf Gesamtmasse MMDI, Nicht-MMDI-Bestandteile allenfalls in Spuren (< 10 ppm) vorhanden;* verkaufsfähiges Produkt. |
| 140-2 | 6,85 | 98,5 % 4,4'-MMDI / 1,5 % 2,4'-MMDI | *Massenprozent bezogen auf Gesamtmasse MMDI, Nicht-MMDI-Bestandteile allenfalls in Spuren (< 10 ppm) vorhanden;* verkaufsfähiges Produkt. |
| 140-3 | **0,14** | **5,6** % 4,4'-MMDI / **65,1** % 2,4'-MMDI / **29,3** % 2,2'-MMDI / | *Massenprozent bezogen auf Gesamtmasse MMDI, Nicht-MMDI-Bestandteile allenfalls in Spuren (< 100 ppm) vorhanden*; fällt als verkaufsfähiges Produkt an (kann alternativ auch zum Beispiel mit Fraktion 141 aus B.II) vermischt werden). |

### Beispiel 3 (erfindungsgemäß - Destillationskolonnen ohne Trennwand)

Es wurde verfahren wie in Beispiel 1 mit dem Unterschied, dass die Nebenkomponenten-Fraktion (150) als Bestandteil *des Zulaufs der Destillationskolonne 2400-6* verwendet wurde. Hierzu wurde der Strom 150 mit dem Destillat der Destillationskolonne 2400-3 vermischt. Dabei wurden folgende Betriebsparameter eingehalten:

**Tabelle 5: Betriebsparameter in Beispiel 3**

| **Destillationskolonne** | **Kopfdruck / mbar_{(abs.)}** | **Sumpftemperatur / °C** | **Kopftemperatur / °C** | **Zulaufmenge / t h⁻¹** |
|---|---|---|---|---|
| 2400-1 | 7,0 | 205 | 196 | 9,80 *(Strom 142)* |
| | | | | 1,00 *(Sumpf aus 2400-4)* |
| 2400- 2 | 6,0 | 212 | 190 | 9,80 *(Destillat aus 2400-1)* |
| 2400-3 | 6,0 | 210 | 188 | 1,95 *(Destillat aus 2400-2)* |
| 2400-4 | 6,0 | 213 | 201 | 1,80 *(Sumpf aus 2400-3)* |
| | | | | 1,00 *(Sumpf aus 2400-5)* |
| 2400-5 | 6,0 | 210 | 197 | 7,85 *(Sumpf aus 2400-2)* |
| 2400-6 | 6,0 | 210 | 161 | 0,15 *(Destillat aus 2400-3)* |
| | | | | **0,035 *(Strom 150)*** |

Die folgenden Ströme verließen die Destillationssequenz:

**Tabelle 6: In Beispiel 3 erhaltene Produktströme**

| **Strom** | **Menge / t h⁻¹** | **Analytik** | **Anmerkung** |
|---|---|---|---|
| 143 | 1,00 | **25,6** Massen-% NCO Viskosität **389** mPa · s | Wird mit Strom 141 aus B.II) vermischt. |
| 140-1 | 1,80 | 44,9 % 4,4'-MMDI / 55,0 % 2,4'-MMDI / 0,1 % 2,2'-MMDI / | *Massenprozent bezogen auf Gesamtmasse MMDI; Nicht-MMDI-Bestandteile allenfalls in Spuren (<10 ppm) vorhanden;* |
| | | | verkaufsfähiges Produkt. |
| 140-2 | 6,85 | 98,5 % 4,4'-MMDI / 1,5 % 2,4'-MMDI | *Massenprozent bezogen auf Gesamtmasse MMDI, Nicht-MMDI-Bestandteile allenfalls in Spuren (< 10 ppm) vorhanden;* |
| | | | verkaufsfähiges Produkt. |
| 140-3 | **0,14** | **7,1** % 4,4'-MMDI / **70,0** % 2,4'-MMDI / **22,9** % 2,2'-MMDI / | *Massenprozent bezogen auf Gesamtmasse MMDI, Nicht-MMDI-Bestandteile allenfalls in Spuren (< 100 ppm) vorhanden;* |
| | | | fällt als verkaufsfähiges Produkt an (kann alternativ auch zum Beispiel mit Fraktion 141 aus B.II) vermischt werden). |

Die Beispiele 1 bis 3 zeigen, dass die erfindungsgemäße Rückführung der Nebenkomponenten-Fraktion (150) eine Erhöhung der Ausbeute an der Reinfraktion 140-3 ermöglicht (weil weniger Isocyanate mit der Nebenkomponenten-Fraktion (150) in die Verbrennung geführt werden), und zwar ohne dass der Nebenkomponentengehalt in irgend einem Wertproduktstrom signifikant ansteigt.

### Beispiel 4 (Vergleichsbeispiel; Destillationskolonne mit Trennwand)

Die Reinigung und Isomerentrennung wurde mit folgender Ausnahme nach einem Verfahren gemäß FIG. 3b durchgeführt:
Die Nebenkomponenten-Fraktion (150) wurde nicht in den Zulauf der Trennwandkolonne zurückgeführt, sondern verbrannt.

Dabei wurden folgende Betriebsparameter eingehalten:

**Tabelle 7: Betriebsparameter in Beispiel 4**

| **Destillationskolonne** | **Kopfdruck** / **mbar_{(abs.)}** | **Sumpftemperatur** / **°C** | **Kopftemperatur** / **°C** | **Zulaufmenge** / **t h⁻¹** |
|---|---|---|---|---|
| 2400-1 | 5,0 | 217 | 179 | 20,5 *(Strom 142)* |
| 2400-2 | 5,0 | 189 | 174 | 3,3 *(oberer Seitenstrom aus 2400-1; dort bei 195 °C entnommen)* |
| 2400-3 | 8,0 | 214 | 204 | 15,7 *(unterer Seitenstrom aus 2400-1; dort bei 200 °C entnommen)* |
| 2400-4 | 10,0 | 215 | 195 | 1,6 *(Gemisch aus den gasförmig abgezogenen Kopffraktionen der Trennwandkolonne (2400-1) und der Seitenabzugskolonne (2410) aus Schritt B.II)* |

Die folgenden Ströme verließen die Destillationssequenz:

**Tabelle 8: In Beispiel 4 erhaltene Produktströme**

| **Strom** | **Menge / t h⁻¹** | **Analytik** | **Anmerkung** |
|---|---|---|---|
| 143 | 1,0 | 26,1 Massen-% NCO Viskosität 390 mPa · s | Wird mit Strom 141 aus B.II) vermischt. |
| 140-1 | 3,3 | 43,8 % 4,4'-MMDI / 56,1 % 2,4'-MMDI / 0,1 % 2,2'-MMDI / | *Massenprozent bezogen auf Gesamtmasse MMDI; Nicht-MMDI-Bestandteile allenfalls in Spuren (< 10 ppm) vorhanden;* |
| | | | verkaufsfähiges Produkt. |
| 140-2 | 15,7 | 98,4 % 4,4'-MMDI / 1,6 % 2,4'-MMDI | *Massenprozent bezogen auf Gesamtmasse MMDI, Nicht-MMDI-Bestandteile allenfalls in Spuren (< 10 ppm) vorhanden;* |
| | | | verkaufsfähiges Produkt. |
| 140-3 | 0,80 | 13,6 % 4,4'-MMDI / 46,4 % 2,4'-MMDI / 40,0 % 2,2'-MMDI | *Massenprozent bezogen auf Gesamtmasse MMDI, Nicht-MMDI-Bestandteile allenfalls in Spuren (< 100 ppm) vorhanden*; |
| | | | grundsätzlich verkaufsfähiges Produkt; wird i. A. zum Beispiel mit Fraktion 141 aus B.II) vermischt). |
| *Seitenabzug von 2400-4* | 0,70 | 3,5 % 4,4'-MMDI / 57,5 % 2,4'-MMDI / 39,0 % 2,2'-MMDI / | *Massenprozent bezogen auf Gesamtmasse MMDI, Nicht-MMDI-Bestandteile allenfalls in Spuren (< <0,1* %) *vorhanden;* |
| | | | Verwendung als Ringflüssigkeit der Vakuumpumpen; kann alternativ auch mit anderen MMDI-Reinfraktionen verschnitten oder als solches weiterverwendet werden. |

**Tabelle 8 (fortgesetzt): In Beispiel 4 erhaltene Produktströme**

| **Strom** | **Menge / t h⁻¹** | **Analytik** | **Anmerkung** |
|---|---|---|---|
| 150 | 0,10 | 2,9 % 2,4'-MMDI / 92,1 % 2,2'-MMDI / 5,0 % Nebenkomponenten (PHI, MCB, Acridinhydrochlorid, etc.) | Wird verbrannt. |

### Beispiel 5 (erfindungsgemäß - Destillationskolonne mit Trennwand; Prozess-Simulation)

Es wird verfahren wie in FIG. 3b gezeigt (d. h., dass im Unterschied zu Beispiel 4 die Nebenkomponenten-Fraktion (150) nicht verbrannt, sondern in den Zulauf der Trennwandkolonne zurückgeführt wird), ansonsten jedoch unter gleichen Betriebsbedingungen wie in Beispiel 4. Die Ausbeute an Strom 140-3 erhöht sich um 0,10 t/h auf 0,90 t/h, wobei die Zusammensetzung des Stromes 140-3 im Wesentlichen gleich bleibt (es werden weniger Isocyanate über die Nebenkomponenten-Fraktion (150) in die Verbrennung geleitet, ohne dass die Produktqualität darunter leidet).

## Patentansprüche

1. Verfahren zur Herstellung von Methylen-Diphenylen-Diisocyanat und optional eines Gemisches aus Methylen-Diphenylen-Diisocyanat und Polymethylen-Polyphenylen-Polyisocyanat, umfassend die Schritte:
α) Bereitstellen einer Methylen-Diphenylen-Diisocyanat und Nebenkomponenten enthaltenden Fraktion mit einem, auf ihre Gesamtmasse bezogenen, per Gaschromatographie bestimmten Massenanteil an Methylen-Diphenylen-Diisocyanat von mehr als 98,0 % (142), optional durch
α.1) Abtrennung von Methylen-Diphenylen-Diisocyanat und Nebenkomponenten aus einer Methylen-Diphenylen-Diisocyanat und Polymethylen-Polyphenylen-Polyisocyanat enthaltenden Fraktion (100) unter Erhalt
(i) eines an Polymethylen-Polyphenylen-Polyisocyanat angereicherten Gemisches aus Methylen-Diphenylen-Diisocyanat und Polymethylen-Polyphenylen-Polyisocyanat (141) und
(ii) der Methylen-Diphenylen-Diisocyanat und Nebenkomponenten enthaltenden Fraktion mit einem, auf ihre Gesamtmasse bezogenen, per Gaschromatographie bestimmten Massenanteil an Methylen-Diphenylen-Diisocyanat von mehr als 98,0 % (142);
β) Reinigung, umfassend eine Isomerentrennung, der Methylen-Diphenylen-Diisocyanat und Nebenkomponenten enthaltenden Fraktion (142) durch Destillation und/oder Kristallisation in zwei oder mehr, bevorzugt in drei bis zehn, besonders bevorzugt in vier bis acht, Teilschritten (a, b, ...) unter Erhalt mindestens von
(i) zwei oder mehr, bevorzugt zwei bis vier, besonders bevorzugt zwei bis drei, Methylen-Diphenylen-Diisocyanat-Reinfraktionen (140-1, 140-2, ...), die jeweils einen, auf ihre Gesamtmasse bezogenen, per Gaschromatographie bestimmten Massenanteil an Methylen-Diphenylen-Diisocyanat von 99,9 % oder mehr aufweisen und
(ii) einer Nebenkomponenten-Fraktion (150) mit einem, auf ihre Gesamtmasse bezogenen, per Gaschromatographie bestimmten Massenanteil an Methylen-Diphenylen-Diisocyanat im Bereich von 20,0 % bis 98,0 %,
**dadurch gekennzeichnet, dass** die in Schritt β) erhaltene Nebenkomponenten-Fraktion (150)
in einen der Teilschritte (a, b,...) von Schritt β), in dem keine der Methylen-Diphenylen-Diisocyanat-Reinfraktionen (140-1, 140-2, ...) aus Schritt β) als Destillat oder Kristallisat gewonnen wird,
und/oder, insofern durchgeführt,
in Schritt α.1)
zurückgeführt wird.

2. Verfahren gemäß Anspruch 1, umfassend den Schritt α.1).

3. Verfahren gemäß Anspruch 2, bei welchem die Methylen-Diphenylen-Diisocyanat und Polymethylen-Polyphenylen-Polyisocyanat enthaltende Fraktion (100) durch folgende Schritte erhalten wird:
A) Umsetzung eines Gemisches (2) aus Methylen-Diphenylen-Diamin und Polymethylen-Polyphenylen-Polyamin mit Phosgen (3) in Gegenwart eines organischen Lösungsmittels (4), wobei Phosgen (3) im stöchiometrischen Überschuss bezogen auf alle vorhandenen primären Aminogruppen eingesetzt wird, unter Erhalt eines flüssigen, Methylen-Diphenylen-Diisocyanat und Polymethylen-Polyphenylen-Polyisocyanat und Nebenkomponenten enthaltenden Stroms (60) und eines gasförmigen, Chlorwasserstoff und Phosgen enthaltenden Stroms (70);
B) Aufarbeitung mindestens des flüssigen, Methylen-Diphenylen-Diisocyanat und Polymethylen-Polyphenylen-Polyisocyanat und Nebenkomponenten enthaltenden Stroms (60), umfassend:
• eine Vorreinigung unter Abtrennung eines ersten Teils der Nebenkomponenten unter Gewinnung der flüssigen, an Nebenkomponenten abgereicherten, Methylen-Diphenylen-Diisocyanat und Polymethylen-Polyphenylen-Polyisocyanat enthaltenden Fraktion (100).

4. Verfahren gemäß Anspruch 3, bei welchem das in Schritt A) eingesetzte organische Lösungsmittel (4) ausgewählt ist aus der Gruppe bestehend aus Monochlorbenzol, Dichlorbenzol, Dioxan, Toluol, Xylol, Methylenchlorid, Perchlorethylen, Trichlorfluormethan und Butylacetat.

5. Verfahren gemäß Anspruch 3 oder 4, bei welchem die Vorreinigung umfasst:
(1) Abtrennung eines Gasstroms (90) enthaltend Chlorwasserstoff und Phosgen aus dem Methylen-Diphenylen-Diisocyanat, Polymethylen-Polyphenylen-Polyisocyanat und Nebenkomponenten enthaltenden Strom (60);
(2) Abtrennung eines Gasstroms (110) enthaltend organisches Lösungsmittel (4) aus der in Schritt (1) nach Abtrennung des Gasstroms (90) enthaltend Chlorwasserstoff und Phosgen verbleibenden Flüssigphase, wobei die flüssige, an Nebenkomponenten abgereicherte, Methylen-Diphenylen-Diisocyanat und Polymethylen-Polyphenylen-Polyisocyanat enthaltende Fraktion (100) erhalten wird;
und optional,
(3) Trennung des Gasstroms (110) enthaltend organisches Lösungsmittel (4) in einen flüssigen Strom (120) enthaltend organisches Lösungsmittel (4) und einen Gasstrom (130) enthaltend Phosgen.

6. Verfahren gemäß einem Ansprüche 3 bis 5, bei welchem in Schritt B) auch der gasförmige, Chlorwasserstoff und Phosgen enthaltende Strom (70) aufgearbeitet wird, wobei diese Aufarbeitung umfasst:
• Abtrennung von Phosgen aus dem gasförmigen, Chlorwasserstoff und Phosgen enthaltenden Strom (70), insbesondere nach Vereinigung mit dem Gasstrom (90) enthaltend Chlorwasserstoff und Phosgen, unter Erhalt eines Gasstroms (170) enthaltend Chlorwasserstoff, wobei, sofern vorhanden, auch der Gasstrom (130) enthaltend Phosgen diesem Phosgenabtrennungsschritt unterzogen wird;
und optional den weiteren Schritt
• Abtrennung von Chlorwasserstoff aus dem Gasstrom (170) enthaltend Chlorwasserstoff.

7. Verfahren gemäß einem der Ansprüche 3 bis 6, bei welchem Schritt β) destillativ durchgeführt wird.

8. Verfahren gemäß Anspruch 7, bei welchem Schritt β) vier bis acht Teilschritte umfasst, wobei jeder Teilschritt einer Destillation in einer Destillationskolonne ohne Trennwand entspricht, wobei die erste Methylen-Diphenylen-Diisocyanat-Reinfraktion (140-1) und die zweite Methylen-Diphenylen-Diisocyanat-Reinfraktion (140-2) in verschiedenen Destillationskolonnen jeweils als Destillat erhalten werden, wobei die Nebenkomponenten-Fraktion (150) in einer Destillationskolonne, die von denen zur Gewinnung der ersten und zweiten Methylen-Diphenylen-Diisocyanat-Reinfraktion verschieden ist, als Destillat gewonnen wird, wobei in dieser Destillationskolonne eine dritte Methylen-Diphenylen-Diisocyanat-Reinfraktion (140-3) als Sumpfprodukt anfällt.

9. Verfahren gemäß Anspruch 8, bei welchem die Nebenkomponenten-Fraktion (150) in den Zulauf der Destillationskolonne, in welcher die Nebenkomponenten-Fraktion (150) erhalten wurde, eingespeist wird.

10. Verfahren gemäß Anspruch 7, bei welchem Schritt β) zwei oder mehr Teilschritte umfasst, wovon mindestens ein Teilschritt in einer Trennwandkolonne durchgeführt wird.

11. Verfahren gemäß Anspruch 10, bei welchem der in Schritt α.1) erhaltene Methylen-Diphenylen-Diisocyanat und Nebenkomponenten enthaltende Strom (142) in Schritt β) in eine Trennwandkolonne geleitet wird, welcher zwei vorgereinigte Methylen-Diphenylen-Diisocyanat-Fraktionen (140-11, 140-22) flüssig als Seitenströme entnommen werden, und welcher ein Nebenkomponenten und Methylen-Diphenylen-Diisocyanat enthaltender Kopfstrom entnommen wird,
wobei die vorgereinigten Methylen-Diphenylen-Diisocyanat-Fraktionen (140-11, 140-22, ...) in weiteren Destillationsstufen zu einer ersten und einer zweiten Methylen-Diphenylen-Diisocyanat-Reinfraktionen (140-1, 140-2) feingereinigt werden,
wobei der Nebenkomponenten und Methylen-Diphenylen-Diisocyanat enthaltende Kopfstrom der Trennwandkolonne in einer Destillationskolonne, die optional als Seitenabzugskolonne mit oder ohne Trennwand ausgestaltet sein kann, unter Erhalt der Nebenkomponenten-Fraktion (150) als Kopfstrom, einer dritten Methylen-Diphenylen-Diisocyanat-Reinfraktion (140-3) als Sumpfstrom und optional einer vierten Methylen-Diphenylen-Diisocyanat-Reinfraktion (140-4) als Seitenstrom destilliert wird,
wobei die Nebenkomponenten-Fraktion (150) in Schritt α.1) oder in die Trennwandkolonne aus Schritt β) zurückgeführt wird.

12. Verfahren gemäß einem der Ansprüche 1 bis 6, bei welchem Schritt β) mindestens einen Teilschritt umfasst, in welchem eine Kristallisation durchgeführt wird, wobei das in der Kristallisation erhaltene Kristallisat eine Methylen-Diphenylen-Diisocyanat-Reinfraktion ist oder durch weitere Reinigung in eine Methylen-Diphenylen-Diisocyanat-Reinfraktion überführt werden kann.

13. Verfahren gemäß Anspruch 12, bei welchem die in dem mindestens einen Teilschritt, in welchem eine Kristallisation durchgeführt wird, anfallende Mutterlauge in mindestens zwei weiteren Teilschritten destilliert wird, wobei mindestens eine weitere Methylen-Diphenylen-Diisocyanat-Reinfraktion und die Nebenkomponenten-Fraktion (150) erhalten werden.

14. Verfahren gemäß Anspruch 13, bei welchem die Mutterlauge in drei weiteren Teilschritten destilliert wird, in welchen zwei Methylen-Diphenylen-Diisocyanat-Reinfraktionen erhalten werden.

## Claims

1. Process for preparing methylene diphenylene diisocyanate and optionally a mixture of methylene diphenylene diisocyanate and polymethylene polyphenylene polyisocyanate, comprising the steps of:
α) providing a fraction comprising methylene diphenylene diisocyanate and secondary components with a proportion by mass of methylene diphenylene diisocyanate, based on its total mass and determined by gas chromatography, of more than 98.0% (142), optionally by
α.1) separating methylene diphenylene diisocyanate and secondary components from a fraction (100) comprising methylene diphenylene diisocyanate and polymethylene polyphenylene polyisocyanate to obtain
(i) a polymethylene polyphenylene polyisocyanate-enriched mixture of methylene diphenylene diisocyanate and polymethylene polyphenylene polyisocyanate (141) and
(ii) the fraction comprising methylene diphenylene diisocyanate and secondary components with a proportion by mass of methylene diphenylene diisocyanate, based on its total mass and determined by gas chromatography, of more than 98.0% (142) ;
β) purifying, comprising an isomer separation, of the fraction (142) comprising methylene diphenylene diisocyanate and secondary components by distillation and/or crystallization in two or more, preferably in three to ten, more preferably in four to eight, partial steps (a, b, ...) to obtain at least
(i) two or more, preferably two to four, more preferably two to three, pure methylene diphenylene diisocyanate fractions (140-1, 140-2, ...), each having a proportion by mass of methylene diphenylene diisocyanate, based on their total mass and determined by gas chromatography, of 99.9% or more and
(ii) a secondary component fraction (150) having a proportion by mass, based on its total mass and determined by gas chromatography, of methylene diphenylene diisocyanate in the range from 20.0% to 98.0%,
**characterized in that** the secondary component fraction (150) obtained in step β) is returned
to one of the partial steps (a, b, ...) of step β) in which none of the pure methylene diphenylene diisocyanate fractions (140-1, 140-2, ...) from step β) is obtained in the form of distillate or crystallizate,
and/or, if conducted,
to step α.1).

2. Process according to Claim 1, comprising step α.1).

3. Process according to Claim 2, in which the fraction (100) comprising methylene diphenylene diisocyanate and polymethylene polyphenylene polyisocyanate is obtained by the following steps:
A) reacting a mixture (2) of methylene diphenylene diamine and polymethylene polyphenylene polyamine with phosgene (3) in the presence of an organic solvent (4), using phosgene (3) in a stoichiometric excess based on all the primary amino groups present, to obtain a liquid stream (60) comprising methylene diphenylene diisocyanate and polymethylene polyphenylene polyisocyanate and secondary components, and a gaseous stream (70) comprising hydrogen chloride and phosgene;
B) working up at least the liquid stream (60) comprising methylene diphenylene diisocyanate and polymethylene polyphenylene polyisocyanate and secondary components, comprising:
• a prepurification to remove a first portion of the secondary components to obtain the liquid fraction (100) depleted of secondary components and comprising methylene diphenylene diisocyanate and polymethylene polyphenylene polyisocyanate.

4. Process according to Claim 3, in which the organic solvent (4) used in step A) is selected from the group consisting of monochlorobenzene, dichlorobenzene, dioxane, toluene, xylene, methylene chloride, perchloroethylene, trichlorofluoromethane and butyl acetate.

5. Process according to Claim 3 or 4, in which the prepurification comprises:
(1) separating a gas stream (90) comprising hydrogen chloride and phosgene from the stream (60) comprising methylene diphenylene diisocyanate, polymethylene polyphenylene polyisocyanate and secondary components;
(2) separating a gas stream (110) comprising organic solvent (4) from the liquid phase remaining in step (1) after separation of the gas stream (90) comprising hydrogen chloride and phosgene to obtain the liquid fraction (100) depleted of secondary components and comprising methylene diphenylene diisocyanate and polymethylene polyphenylene polyisocyanate;
and optionally
(3) separating the gas stream (110) comprising organic solvent (4) into a liquid stream (120) comprising organic solvent (4) and a gas stream (130) comprising phosgene.

6. Process according to any of Claims 3 to 5, in which, in step B), the gaseous stream (70) comprising hydrogen chloride and phosgene is also worked up, where this workup comprises:
• separating phosgene from the gaseous stream (70) comprising hydrogen chloride and phosgene, especially after combining with the gas stream (90) comprising hydrogen chloride and phosgene, to obtain a gas stream (170) comprising hydrogen chloride, where, if present, the gas stream (130) comprising phosgene is also subjected to this phosgene separation step;
and optionally the further step of
• separating hydrogen chloride from the gas stream (170) comprising hydrogen chloride.

7. Process according to any of Claims 3 to 6, in which step β) is performed by distillation.

8. Process according to Claim 7, in which step β) comprises four to eight partial steps, wherein each partial step corresponds to a distillation in a distillation column without a dividing wall, wherein the first pure methylene diphenylene diisocyanate fraction (140-1) and the second pure methylene diphenylene diisocyanate fraction (140-2) are each obtained as distillate in different distillation columns, wherein the secondary component fraction (150) is obtained as distillate in a distillation column other than that for obtaining the first and second pure methylene diphenylene diisocyanate fraction, wherein a third pure methylene diphenylene diisocyanate fraction (140-3) is obtained as bottom product in this distillation column.

9. Process according to Claim 8, in which the secondary component fraction (150) is fed into the feed of the distillation column in which the secondary component fraction (150) has been obtained.

10. Process according to Claim 7, in which step β) comprises two or more partial steps, of which at least one partial step is performed in a dividing wall column.

11. Process according to Claim 10, in which the stream (142) which comprises methylene diphenylene diisocyanate and secondary components and is obtained in step α.1) is passed, in step β), into a dividing wall column from which two prepurified methylene diphenylene diisocyanate fractions (140-11, 140-22) are withdrawn as side streams in liquid form, and from which a top stream comprising secondary components and methylene diphenylene diisocyanate is withdrawn,
wherein the prepurified methylene diphenylene diisocyanate fractions (140-11, 140-22, ...) are subjected to fine purification in further distillation stages to give a first and a second pure methylene diphenylene diisocyanate fraction (140-1, 140-2),
wherein the top stream from the dividing wall column that comprises secondary components and methylene diphenylene diisocyanate is distilled in a distillation column, which may optionally be configured as a side draw column with or without dividing wall, to obtain the secondary component fraction (150) as top stream, a third pure methylene diphenylene diisocyanate fraction (140-3) as bottom stream, and optionally a fourth pure methylene diphenylene diisocyanate fraction (140-4) as side stream,
wherein the secondary component fraction (150) is recycled into step α.1) or into the dividing wall column from step β).

12. Process according to any of Claims 1 to 6, in which step β) comprises at least one partial step in which a crystallization is performed, wherein the crystallizate obtained in the crystallization is a pure methylene diphenylene diisocyanate fraction or can be converted to a pure methylene diphenylene diisocyanate fraction by further purification.

13. Process according to Claim 12, in which the mother liquor obtained in the at least one partial step in which a crystallization is performed is distilled in at least two further partial steps, wherein at least one further pure methylene diphenylene diisocyanate fraction and the secondary component fraction (150) are obtained.

14. Process according to Claim 13, in which the mother liquor is distilled in three further partial steps in which two pure methylene diphenylene diisocyanate fractions are obtained.

## Revendications

1. Procédé pour la préparation de diisocyanate de méthylène-diphénylène et éventuellement d'un mélange de diisocyanate de méthylène-diphénylène et de polyisocyanate de polyméthylène-polyphénylène, comprenant les étapes de :
α) mise à disposition d'une fraction (142) contenant du diisocyanate de méthylène-diphénylène et des composants secondaires présentant une proportion massique de diisocyanate de méthylène-diphénylène de plus de 98,0% par rapport à sa masse totale, déterminée par chromatographie en phase gazeuse, éventuellement par
α.1) séparation de diisocyanate de méthylène-diphénylène et de composants secondaires d'une fraction (100) contenant du diisocyanate de méthylène-diphénylène et du polyisocyanate de polyméthylène-polyphénylène avec obtention
(i) d'un mélange (141) de diisocyanate de méthylène-diphénylène et de polyisocyanate de polyméthylène-polyphénylène enrichi en polyisocyanate de polyméthylène-polyphénylène et
(ii) de la fraction (142) contenant du diisocyanate de méthylène-diphénylène et des composants secondaires présentant une proportion massique de diisocyanate de méthylène-diphénylène de plus de 98,0% par rapport à sa masse totale, déterminée par chromatographie en phase gazeuse ;
β) purification, comprenant une séparation d'isomères, de la fraction (142) contenant du diisocyanate de méthylène-diphénylène et des composants secondaires par distillation et/ou cristallisation en deux étapes partielles ou plus, de préférence en trois à dix étapes partielles, de manière particulièrement préférée en quatre à huit étapes partielles (a, b, ...) avec obtention d'au moins
(i) deux fractions pures ou plus, de préférence deux à quatre fractions pures, de manière particulièrement préférée deux à trois fractions pures (140-1, 140-2, ...) de diisocyanate de méthylène-diphénylène, qui présentent à chaque fois une proportion massique de diisocyanate de méthylène-diphénylène de 99,9% ou plus par rapport à sa masse totale, déterminée par chromatographie en phase gazeuse, et
(ii) une fraction (150) de composants secondaires présentant une proportion massique de diisocyanate de méthylène-diphénylène dans la plage de 20,0% à 98,0% par rapport à sa masse totale, déterminée par chromatographie en phase gazeuse,
**caractérisé en ce que** la fraction (150) de composants secondaires obtenue dans l'étape β) est recyclée dans l'une des étapes partielles (a, b, ...) de l'étape β), dans laquelle n'est obtenue aucune des fractions pures (140-1, 140-2, ...) de diisocyanate de méthylène-diphénylène de l'étape β) sous forme de distillat ou de produit de cristallisation et/ou, le cas échéant, dans l'étape α.1).

2. Procédé selon la revendication 1, comprenant l'étape α.1).

3. Procédé selon la revendication 2, dans lequel la fraction (100) contenant du diisocyanate de méthylène-diphénylène et du polyisocyanate de polyméthylène-polyphénylène est obtenue par les étapes suivantes :
A) transformation d'un mélange (2) de méthylène-diphénylène-diamine et de polyméthylène-polyphénylène-polyamine avec du phosgène (3) en présence d'un solvant organique (4), le phosgène (3) étant utilisé en excès stoechiométrique par rapport à tous les groupes amino primaire présents, avec obtention d'un flux liquide (60), contenant du diisocyanate de méthylène-diphénylène et du polyisocyanate de polyméthylène-polyphénylène et des composants secondaires et d'un flux gazeux (70) contenant de l'acide chlorhydrique et du phosgène ;
B) traitement au moins du flux liquide (60) contenant du diisocyanate de méthylène-diphénylène et du polyisocyanate de polyméthylène-polyphénylène et des composants secondaires, comprenant :
- une prépurification avec séparation d'une première partie des composants secondaires avec obtention de la fraction liquide (100) contenant du diisocyanate de méthylène-diphénylène et du polyisocyanate de polyméthylène-polyphénylène, appauvrie en composants secondaires.

4. Procédé selon la revendication 3, dans lequel le solvant organique (4) utilisé dans l'étape A) est choisi dans le groupe constitué par le monochlorobenzène, le dichlorobenzène, le dioxane, le toluène, le xylène, le chlorure de méthylène, le perchloroéthylène, le trichlorofluorométhane et l'acétate de butyle.

5. Procédé selon la revendication 3 ou 4, dans lequel la prépurification comprend :
(1) la séparation d'un flux gazeux (90) contenant de l'acide chlorhydrique et du phosgène à partir du flux (60) contenant du diisocyanate de méthylène-diphénylène, du polyisocyanate de polyméthylène-polyphénylène et des composants secondaires ;
(2) la séparation d'un flux gazeux (110) contenant du solvant organique (4) à partir de la phase liquide restant dans l'étape (1) après la séparation du flux gazeux (90) contenant de l'acide chlorhydrique et du phosgène, la fraction liquide (100) contenant du diisocyanate de méthylène-diphénylène et du polyisocyanate de polyméthylène-polyphénylène, appauvrie en composants secondaires étant obtenue ;
et éventuellement
(3) la séparation du flux gazeux (110) contenant du solvant organique (4) en un flux liquide (120) contenant du solvant organique (4) et un flux gazeux (130) contenant du phosgène.

6. Procédé selon l'une quelconque des revendications 3 à 5, dans lequel, dans l'étape B), le flux gazeux (70) contenant de l'acide chlorhydrique et du phosgène est également traité, ce traitement comprenant :
- la séparation de phosgène à partir du flux gazeux (70) contenant de l'acide chlorhydrique et du phosgène, en particulier après réunion avec le flux gazeux (90) contenant de l'acide chlorhydrique et du phosgène, avec obtention d'un flux gazeux (170) contenant de l'acide chlorhydrique où, le cas échéant, le flux gazeux (130) contenant du phosgène est également soumis à cette étape de séparation de phosgène ;
éventuellement l'étape supplémentaire de
- séparation d'acide chlorhydrique du flux gazeux (170) contenant de l'acide chlorhydrique.

7. Procédé selon l'une quelconque des revendications 3 à 6, dans lequel l'étape β) est réalisée par distillation.

8. Procédé selon la revendication 7, dans lequel l'étape β) comprend quatre à huit étapes partielles, chaque étape partielle correspondant à une distillation dans une colonne de distillation sans paroi de séparation, la première fraction pure (140-1) de diisocyanate de méthylène-diphénylène et la deuxième fraction pure (140-2) de diisocyanate de méthylène-diphénylène étant obtenues à chaque fois sous forme de distillat dans des colonnes de distillation différentes, la fraction (150) de composants secondaires étant obtenue sous forme de distillat dans une colonne de distillation, qui est différente de celle pour l'obtention de la première et de la deuxième fraction pure de diisocyanate de méthylène-diphénylène, une troisième fraction pure (140-3) de diisocyanate de méthylène-diphénylène étant produite sous forme de produit de fond dans cette colonne de distillation.

9. Procédé selon la revendication 8, dans lequel la fraction (150) de composants secondaires est injectée dans l'alimentation de la colonne de distillation dans laquelle la fraction (150) de composants secondaires a été obtenue.

10. Procédé selon la revendication 7, dans lequel l'étape β) comprend deux étapes partielles ou plus dont au moins une étape partielle est réalisée dans une colonne à paroi de séparation.

11. Procédé selon la revendication 10, dans lequel le flux (142) contenant du diisocyanate de méthylène-diphénylène et des composants secondaires obtenu dans l'étape α.1) est guidé dans l'étape β) dans une colonne à paroi de séparation dont on prélève deux fractions prépurifiées (140-11, 140-22) de diisocyanate de méthylène-diphénylène sous forme liquide en tant que flux latéraux et dont on prélève un flux de tête contenant des composants secondaires et du diisocyanate de méthylène-diphénylène,
les fractions prépurifiées (140-11, 140-22, ...) de diisocyanate de méthylène-diphénylène étant purifiées de manière plus fine dans d'autres étages de distillation en une première et une deuxième fraction pure (140-1, 140-2) de diisocyanate de méthylène-diphénylène,
le flux de tête contenant des composants secondaires et du diisocyanate de méthylène-diphénylène de la colonne à paroi de séparation étant distillé dans une colonne de distillation, qui peut éventuellement être conçue comme colonne à soutirage latéral avec ou sans paroi de séparation, avec obtention de la fraction (150) de composants secondaires en tant que flux de tête, d'une troisième fraction pure (140-3) de diisocyanate de méthylène-diphénylène en tant que flux de fond et éventuellement d'une quatrième fraction pure (140-4) de diisocyanate de méthylène-diphénylène en tant que flux latéral,
la fraction (150) de composants secondaires étant recyclée dans l'étape (α.1) ou dans la colonne à paroi de séparation de l'étape β).

12. Procédé selon l'une quelconque des revendications 1 à 6, dans lequel l'étape β) comprend au moins une étape partielle, dans laquelle une cristallisation est réalisée, le produit de cristallisation obtenu dans la cristallisation étant une fraction pure de diisocyanate de méthylène-diphénylène ou pouvant être transformé en une fraction pure de diisocyanate de méthylène-diphénylène par une nouvelle purification.

13. Procédé selon la revendication 12, dans lequel la lessive-mère produite dans ladite au moins une étape partielle dans laquelle une cristallisation est réalisée est distillée dans au moins deux autres étapes partielles, au moins une autre fraction pure de diisocyanate de méthylène-diphénylène et la fraction (150) de composants secondaires étant obtenues.

14. Procédé selon la revendication 13, dans lequel la lessive-mère est distillée dans trois autres étapes partielles, dans lesquelles deux fractions pures de diisocyanate de méthylène-diphénylène sont obtenues.
